# EUROPEAN PATENT SPECIFICATION

(11) **EP 2 997 955 B1**
(45) Date of publication and mention of the grant of the patent: **01.03.2017**
(21) Application number: 14797023.0
(22) Date of filing: 07.05.2014
(51) Int. Cl.: A61Q 17/04, A61Q 1/02, A61Q 19/00, A61K 8/02, A61K 8/06, A61K 8/27, A61K 8/29, A61K 8/31, A61K 8/35, A61K 8/81

(54) **OIL-IN-WATER COSMETIC**
ÖL-IN-WASSER-KOSMETIKUM
PRODUIT COSMÉTIQUE TYPE HUILE DANS L'EAU

(30) Priority: 15.05.2013 JP 2013103486
(43) Date of publication of application: 23.03.2016
(73) Proprietor: FUJIFILM Corporation, Tokyo 106-8620 (JP)
(72) Inventor: AOKI, Minako, Ashigarakami-gun Kanagawa 258-8577 (JP); SAKAGUCHI, Hiroyuki, Ashigarakami-gun Kanagawa 258-8577 (JP)
(74) Representative: Klunker . Schmitt-Nilson . Hirsch
(86) International application number: PCT/JP2014/062280
(87) International publication number: WO 2014/185315

(56) References cited:
- EP-A2- 1 803 435
- WO-A1-2010/098249
- WO-A1-2012/102296
- WO-A1-2013/002278
- WO-A1-2013/065643
- WO-A1-2013/065644
- WO-A2-2012/058675
- JP-A- 2011 506 703
- US-A1- 2011 129 509
- DATABASE WPI Week 200455 Thomson Scientific, London, GB; AN 2004-565988 XP002754883, & JP 2004 217534 A (SHISEIDO CO LTD) 5 August 2004 (2004-08-05)

## Description

### BACKGROUND OF THE INVENTION

### 1. Field of the Invention

The present invention relates to an oil-in-water cosmetic.

### 2. Description of the Related Art

Thus far, a variety of organic ultraviolet absorbents have been used to impart an ultraviolet ray shielding effect to cosmetics, and particularly, ultraviolet ray shielding powder obtained by complexing an ultraviolet absorbent which is an organic compound with inorganic powder has been known.

For example, WO2010/098249A discloses complex powder obtained by coating the surface of powder serving as a base material with an organic compound having an ultraviolet ray absorbing function. In WO2010/098249A, it is described that this complex powder is obtained by complexing an organic compound having an ultraviolet ray absorbing function and an inorganic colorant having ultraviolet ray scattering function, is powder that can be stably formulated into cosmetics, and is an organic ultraviolet absorbent capable of maintaining a strong ultraviolet ray absorbing effect and having favorable dispersibility. WO2010/098249A discloses complex powder obtained by coating the surface of a titanium oxide fine particle with butyl methoxy dibenzoyl methane as the above-described complex powder.

Meanwhile, as a natural component capable of exhibiting a variety of functions, a number of cosmetics containing a carotenoid are known. Since a carotenoid has properties of being easily decomposed by light, it is required to stably add a carotenoid to a cosmetic.

For example, JP2012-211114A discloses a water-in-oil emulsified cosmetic into which hydrophobilized inorganic powder such as dimethyl polysiloxane-treated titanium oxide, a specific silicone-based surfactant, silicone oil, triglyceride, and astaxanthin are formulated. It is described that this water-in-oil emulsified cosmetic is well spread even when formulated with powder and has excellent temporal stability of astaxanthin even when coexisting with a powder component.

### SUMMARY OF THE INVENTION

However, when powder having butyl methoxy benzoyl methane on the surface is formulated into an emulsified substance, there are cases in which feelings during use such as a fresh feeling when being applied and a greasy feeling after being applied are impaired. In addition, there is still room for improving the temporal stability of carotenoids in the presence of powder having butyl methoxy benzoyl methane on the surface. As a result, there is an increasing demand for having an excellent feeling during use and satisfying both a favorable ultraviolet ray shielding function and a favorable temporal stability of carotenoids.

An object of the present invention is to provide an oil-in-water cosmetic having a favorable ultraviolet ray shielding effect, an excellent temporal stability of carotenoids, and a favorable feeling during use.

The present invention is as described below.
[1] An oil-in-water cosmetic including: surface-treated powder including at least one oxide selected from a group consisting of titanium oxide and zinc oxide and having 4-tert-butyl-4-methoxy benzoyl methane on a surface; at least one carotenoid; and at least one copolymer including acryloyldimethyl taurate as a copolymerization component.
[2] The oil-in-water cosmetic according to [1], further including: at least one compound selected from a group consisting of a sorbitan fatty acid ester, a glycerin fatty acid ester, and a fatty acid.
[3] The oil-in-water cosmetic according to [1], further including: at least one sorbitan fatty acid ester.
[4] The oil-in-water cosmetic according to any one of [1] to [3], in which the copolymer including acryloyldimethyl taurate as a copolymerization component is at least one copolymer selected from a group consisting of an ammonium acryloyldimethyl taurate/vinyl pyrrolidone copolymer, a sodium acrylate/sodium acryloyldimethyl taurate copolymer, and an ammonium acryloyldimethyl taurate/beheneth-25 methacrylate crosspolymer.
[5] The oil-in-water cosmetic according to any one of [1] to [4], in which the surface-treated powder is included in an oil phase.
[6] The oil-in-water cosmetic according to [5], further including: at least one lipophilic thickener, in which the at least one lipophilic thickener is included in the oil phase including the surface-treated powder.
[7] The oil-in-water cosmetic according to [6], in which the lipophilic thickener is at least one lipophilic thickener selected from a group consisting of nylon-12, a dimethicone/vinyl dimethincone crosspolymer, polymethylsilsesquioxane, a vinyl dimethicone/methylsilsesquioxane crosspolymer, and an alkyl acrylate/dimethicone copolymer.
[8] The oil-in-water cosmetic according to [6] or [7], in which a total content of the liphophilic thickener is in a range of 0.01% by mass to 9% by mass.
[9] The oil-in-water cosmetic according to any one of [1] to [4], in which the surface-treated powder is included in a water phase.

According to the present invention, it is possible to provide an oil-in-water cosmetic having a favorable ultraviolet ray shielding effect, an excellent temporal stability of carotenoids, and a favorable feeling during use.

### DESCRIPTION OF THE PREFERRED EMBODIMENTS

An oil-in-water cosmetic of the present invention includes (a) surface-treated powder including at least one oxide selected from a group consisting of titanium oxide and zinc oxide and having 4-tert-butyl-4-methoxy benzoyl methane on a surface, (b) at least one carotenoid, and (c) at least one copolymer including acryloyldimethyl taurate as a copolymerization component.

Since the oil-in-water cosmetic of the present invention includes a combination of predetermined surface-treated powder having 4-tert-butyl-4-methoxy benzoyl methane on the surface and a copolymer including acryloyldimethyl taurate as a copolymerization component, a favorable ultraviolet ray shielding effect, an excellent temporal stability of carotenoids, and a favorable feeling during use can be obtained.

That is, it has been found that the specific surface-treated powder having 4-tert-butyl-4-methoxy benzoyl methane on the surface is a surface-treated powder having a favorable ultraviolet ray shielding function and contributes to improvement in the temporal stability of carotenoids. In addition, when the copolymer including acryloyldimethyl taurate as a copolymerization component is formulated with the oil-in-water cosmetic, it is assumed that the dispersibility of the specific surface-treated powder improves, accordingly, the feeling of the oil-in-water cosmetic during use improves, and additionally, the temporal stability of carotenoids further improves. However, the present invention is not confined by any specific theories.

Generally, the surface of powder is silicone-treated or silica-treated using hydrogen dimethicone. In contrast, the surface-treated powder in the present invention has 4-tert-butyl-4-methoxy benzoyl methane having a hydrocarbon group on the surface. In addition, the copolymer including acryloyldimethyl taurate as a copolymerization component, which is used in the present invention, is also a component having a hydrocarbon group. Based on the above-described facts, in the present invention, it is assumed that the copolymer including acryloyldimethyl taurate as a copolymerization component interacts with 4-tert-butyl-4-methoxy benzoyl methane on the surface of the surface-treated powder and is thus present in a state of being evenly dispersed in the oil-in-water cosmetic. As a result, in the oil-in-water cosmetic of the present invention, it is considered that aggregation of the surface-treated powder is suppressed and a favorable feeling during use can be imparted. However, the present invention is not confined by any specific theories.

In the present specification, the term "step" refers not only to an independent step but also to a step which cannot be clearly distinguished from other steps as long as a desired effect of the step can be achieved.

In the present invention, a numerical range expressed using "to" refers to a range including the numerical value before and after the "to" as the upper limit value and the lower limit value respectively.

In the present specification, the amount of individual components in a composition refers to, in a case in which the composition includes a plurality of substances which correspond to the individual components, the total amount of the plurality of substances in the composition unless particularly otherwise described.

In the present specification, an "oil-phase component" refers to a component which can be used to prepare an oil-phase composition by being combined with an oil solution when preparing the oil-in-water cosmetic and can be present in a state of being dispersed or dissolved in an oil droplet of the oil-in-water cosmetic.

In the present specification, a "water-phase component" refers to a component which can be used to prepare a water-phase composition by being combined with an aqueous medium such as water when preparing the oil-in-water cosmetic and can be present in a state of being dispersed or dissolved in a water phase of the oil-in-water cosmetic (an oil droplet is not included).

Hereinafter, the present invention will be described.

The oil-in-water cosmetic of the present invention includes surface-treated powder including at least one oxide selected from a group consisting of titanium oxide and zinc oxide and having 4-tert-butyl-4-methoxy benzoyl methane on the surface, at least one carotenoid, and at least one copolymer including acryloyldimethyl taurate as a copolymerization component and includes other components as necessary.

### (a) Surface-treated powder

The surface-treated powder in the present invention is surface-treated powder including at least one oxide selected from a group consisting of titanium oxide (TiO₂) and zinc oxide (ZnO) and having 4-tert-butyl-4-methoxy benzoyl methane on the surface (hereinafter, in some cases, simply referred to as "surface-treated powder"). Since the surface-treated powder includes 4-tert-butyl-4-methoxy benzoyl methane, the surface-treated powder has a favorable ultraviolet ray shielding function.

The surface-treated powder includes at least one oxide selected from a group consisting of titanium oxide and zinc oxide and, from the viewpoint of an ultraviolet ray protecting effect obtained from a difference in the ultraviolet ray shielding wavelength range from 4-tert-butyl-4-methoxy benzoyl methane, the surface-treated powder is preferably titanium oxide. In the present specification, in some cases, at least one oxide selected from a group consisting of titanium oxide and zinc oxide will be collectively referred to as "inorganic powder".

The crystal form of the titanium oxide may be any of anatase, rutile, and brookite. The crystal form of the titanium oxide is preferably rutile. In a case in which the crystal form of the titanium oxide is a rutile form, it is possible to more effectively shield ultraviolet rays, which is preferable.

The zinc oxide preferably has a wurtzite-form crystal structure.

Any inorganic powder which can be generally used for cosmetic products can be used without any particular limitations depending on the shape such as a spherical shape, a plate shape, or a needle shape, the particle diameter such as a haze form, fine particles, or a colorant class, the particle structure such as a porous structure or a pore-free structure.

From the viewpoint of the ultraviolet ray shielding function, the average primary particle diameter of the inorganic powder is preferably 200 nm or smaller. From the viewpoint of the transparency and the feeling during use of the cosmetic, the average primary particle diameter of the inorganic powder is preferably in a range of 1 nm to 90 nm and more preferably in a range of 5 nm to 50 nm. As the average primary particle diameter of the inorganic powder, the average value obtained by dispersing the inorganic powder, then, capturing an image of 1000 or more powder particles using a transmission electron microscope, carrying out an imaging process on individual particles in the captured image using an image analysis-type particle size distribution analyzer, and measuring the circle-equivalent diameters of the particles is used. In a case in which a commercially available product is used, the average primary particle diameter of the inorganic powder used in the commercially available product can be applied with no changes as the average primary particle diameter of the inorganic powder.

As the inorganic powder, it is possible to use, for example, inorganic powder having a surface treated with an inorganic substance such as aluminum oxide, silicon oxide, or zirconium oxide, inorganic powder having a surface treated with an organic substance such as stearic acid in order to suppress the surface activity of the inorganic powder. As the inorganic powder, it is possible to use inorganic powder obtained by further carrying out a hydrophobilization treatment such as a silicone treatment, a fatty acid treatment, a metal soap treatment, or a fluorine treatment or a hydrophilization treatment such as a polyacrylic acid treatment on the inorganic powder having a surface treated with an inorganic substance or the inorganic powder having a surface treated with an organic substance.

The surface-treated powder has 4-tert-butyl-4-methoxy benzoyl methane on the surface. The surface-treated powder preferably has 4-tert-butyl-4-methoxy benzoyl methane on the entire outermost surface. However, on the outermost surface of the surface-treated powder, there may be a portion having 4-tert-butyl-4-methoxy benzoyl methane as long as the effect of the present invention is not impaired. Regarding the bonding pattern between the surface of the surface-treated powder (in a case in which the surface of the inorganic powder is further treated with an inorganic substance or an organic substance, the inorganic substance or the organic substance disposed on the surface) and 4-tert-butyl-4-methoxy benzoyl methane, there is no particular limitation as long as 4-tert-butyl-4-methoxy benzoyl methane and the inorganic powder integrally behave. The bonding pattern may be a chemical bond such as a covalent bond or a non-chemical bond such as adsorption.

The presence of 4-tert-butyl-4-methoxy benzoyl methane on the surface of the surface-treated powder can be confirmed by adding ethanol to the surface-treated powder, stirring ethanol and the surface-treated powder together, and measuring the absorbance of a supernatant solution at a wavelength of 355 nm using a spectrophotometer.

The average particle diameter of the surface-treated powder is preferably smaller than 1 µm. When the average particle diameter of the surface-treated powder is smaller than 1 µm, it is considered that the cosmetic being colored by the surface-treated powder is suppressed and a so-called white-turbidity phenomenon tends not to occur. From the viewpoint of the ultraviolet ray shielding function and the feeling during use, the average particle diameter of the surface-treated powder is more preferably in a range of 1 nm to 500 nm and still more preferably in a range of 3 nm to 100 nm.

The average particle diameter of the surface-treated powder can be measured using the same method as for the average primary particle diameter.

Examples of the surface-treated powder include the surface-treated powder disclosed in WO2010/098249A. As the surface-treated powder, it is possible to use HXMT-100ZA (manufactured by TAYCA Corporation), HXMT-10EXA (manufactured by TAYCA Corporation), HXLT-02 (manufactured by TAYCA Corporation), which can be procured from commercially available products.

In the oil-in-water cosmetic of the present invention, the content of the surface-treated powder is not particularly limited, but is preferably in a range of 0.1% by mass to 30% by mass. When the content of the surface-treated powder being used is in the above-described range, it is possible to obtain an oil-in-water cosmetic in which the feeling during use such as a feeling of the spreading of the cosmetic when applying the cosmetic, the temporal stability of the oil-in-water cosmetic, and the temporal stability of carotenoids are more favorable.

From the viewpoint of the feeling during use and the temporal stability of carotenoids, the content of the surface-treated powder is preferably in a range of 1% by mass to 20% by mass and more preferably in a range of 3% by mass to 10% by mass.

From the viewpoint of the temporal stability of carotenoids, the content of the surface-treated powder to the content of the carotenoid in terms of mass is preferably in a range of 1 time to 400000 times, more preferably in a range of 10 times to 40000 times, and still more preferably in a range of 100 times to 4000 times. When the content of the surface-treated powder to the content of the carotenoid in terms of mass is 400000 times or lower, there is a tendency that the temporal stability of carotenoids is further improved.

The surface-treated powder is included in an oil phase or a water phase in the oil-in-water cosmetic. From the viewpoint of the feeling of the cosmetic during use, the surface-treated powder is preferably included in an oil phase.

The surface-treated powder being included in an oil phase means that the surface-treated powder is present in a state of being dispersed in an oil droplet of the oil-in-water cosmetic. The surface-treated powder being included in a water phase means that the surface-treated powder is present in a state of being dispersed in a water phase of the oil-in-water cosmetic.

In a case in which the oil-in-water cosmetic includes the surface-treated powder in an oil phase, for example, it is also possible to prepare a slurry including the surface-treated powder using a volatile oil component such as a silicone oil as a dispersion medium and formulate the surface-treated powder with other components in a slurry form.

The content of the surface-treated powder in the slurry is not particularly limited; however, generally, the content thereof is preferably set in a range of 10% by mass to 80% by mass and more preferably set in a range of 20% by mass to 60% by mass of the total mass of the slurry. When the content of the surface-treated powder in the slurry is 10% by mass or higher of the total mass of the slurry, it is possible to more stably formulate the surface-treated powder with the oil-in-water cosmetic, which is preferable. When the content of the surface-treated powder in the slurry is in a range of 20% by mass to 60% by mass of the total mass of the slurry, it is possible to more stably formulate the surface-treated powder with the oil-in-water cosmetic, which is preferable.

### (b) Carotenoid

The oil-in-water cosmetic of the present invention includes at least one carotenoid. Carotenoids refer to pigments of yellow to red terpenoids, and examples thereof include carotenoids derived from plants, algae, and bacteria. The carotenoids are not limited to naturally-occurring carotenoids and may be any carotenoids obtained according to an ordinary method.

Specific examples of the carotenoid in the present invention include lycopene, α-carotene, β-carotene, γ-carotene, δ-carotene, actinioerythrol, bixin, canthaxanthin, capsorubin, β-8'-apo-carotenal (apocarotenal), β-12'-apo-carotenal, xanthophylls (for example, astaxanthin, fucoxanthin, lutein, zeaxanthin, capsanthin, β-cryptoxanthin, violaxanthin, violerythrin), and hydroxyl derivatives or carboxyl derivatives thereof. Only one carotenoid may be used or a combination of two or more carotenoids may be used. Among these, astaxanthin and lycopene are preferred, and particularly, astaxanthin having an anti-aging effect, an anti-inflammatory effect, and a whitening effect is preferred.

From the viewpoint of efficient development of the functions of the carotenoid, favorable compatibility with the skin, and efficient obtainment of a white-turbidity-preventing effect, the total content of the carotenoid in the oil-in-water cosmetic is preferably in a range of 0.000001% by mass to 1% by mass, more preferably in a range of 0.00001% by mass to 0.1% by mass, and still more preferably in a range of 0.0001% by mass to 0.05% by mass.

### (c) Copolymer including acryloyldimethyl taurate as copolymerization component

The oil-in-water cosmetic of the present invention includes at least one copolymer including acryloyldimethyl taurate as a copolymerization component. The copolymer including acryloyldimethyl taurate as a copolymerization component improves the dispersibility of the surface-treated powder so as to improve the feeling of the oil-in-water cosmetic during use and further strengthens the effect of improving the temporal stability of carotenoids obtained from the surface-treated powder.

Examples of the acryloyldimethyl taurate as a copolymerization component include inorganic salts or organic salts of acryloyldimethyl taurine. Examples of the inorganic salts include a lithium salt, a sodium salt, a potassium salt, a magnesium salt, a calcium salt, an aluminum salt, and an ammonium salt. Examples of the organic salts include a monoalkyl ammonium salt, a dialkyl ammonium salt, a trialkyl ammonium salt, and a tetraalkyl ammonium salt. Examples of an alkyl substituent for amines include an alkyl group having 1 to 22 carbon atoms and a hydroxyalkyl group having 2 to 10 carbon atoms.

Examples of other copolymerization components capable of constituting a copolymer with the acryloyldimethyl taurate include N-vinylpyrrolidone (VP); inorganic salts of acrylic acid and methacrylic acid, for example, a sodium salt and an ammonium salt; methacrylic acid esters of a polyoxyethylene behenyl ether, for example, esters of beheneth-5, beheneth-10, beheneth-15, or beheneth-25.

From the viewpoint of the salt resistance of the cosmetic, the copolymer including acryloyldimethyl taurate as a copolymerization component is preferably at least one copolymer selected from a group consisting of an ammonium acryloyldimethyl taurate/vinyl pyrrolidone copolymer, a sodium acrylate/sodium acryloyldimethyl taurate copolymer, and an ammonium acryloyldimethyl taurate/beheneth-25 methacrylate crosspolymer.

From the viewpoint of the temporal stability of carotenoids and the feeling during use, the total content of the copolymer including the acryloyldimethyl taurate as a copolymerization component is preferably in a range of 0.01% by mass to 8% by mass, more preferably in a range of 0.05% by mass to 5% by mass, and still more preferably in a range of 0.1% by mass to 3% by mass of the total mass of the cosmetic in terms of the amount of a polymer (pure component). When the total content of the copolymer including the acryloyldimethyl taurate as a copolymerization component is in the above-described range, there is a tendency that the temporal stability of carotenoids and spreading of the cosmetic when being applied to the skin are favorable, and the applied cosmetic is felt to be fresh and has excellent compatibility with the skin.

### (d) At least one compound selected from group consisting of sorbitan fatty acid ester, glycerin fatty acid ester, and fatty acid

The oil-in-water cosmetic of the present invention preferably includes at least one compound selected from a group consisting of a sorbitan fatty acid ester, a glycerin fatty acid ester, and a fatty acid (hereinafter, in some cases, collectively referred to as "dispersing agent component"). When the oil-in-water cosmetic includes the dispersing agent component, the dispersibility of the surface-treated powder in the oil-in-water cosmetic further improves. Only one dispersing agent component may be used or a combination of two or more dispersing agent components may be used. From the viewpoint of improving the dispersibility, the oil-in-water cosmetic of the present invention preferably includes a sorbitan fatty acid ester as the dispersing agent component.

### (Sorbitan fatty acid ester)

The sorbitan fatty acid ester is preferably a sorbitan fatty acid ester in which the fatty acid has 8 or more carbon atoms and more preferably a sorbitan fatty acid ester in which the fatty acid has 12 or more carbon atoms.

Examples of the sorbitan fatty acid ester include sorbitan monolaurate, sorbitan monopalmitate, sorbitan monostearate, sorbitan monooleate, sorbitan monoisostearate, sorbitan sesquicaprylate, sorbitan sesquioleate, sorbitan sesquistearate, sorbitan sesquiisostearate, sorbitan trioleate, sorbitan tristearate, sorbitan triisostearate, diglycerol sorbitan tetra-2-ethylhexanoate, diglycerol sorbitan penta-2-ethylhexanoate, and sorbitan cocoate. In the present invention, it is possible to use only one sorbitan fatty acid ester or a combination of two or more sorbitan fatty acid esters. From the viewpoint of the temporal stability of carotenoids and the feeling during use, the sorbitan fatty acid ester is particularly preferably at least one sorbitan fatty acid ester selected from a group consisting of sorbitan monooleate, sorbitan monoisostearate, sorbitan sesquioleate, and sorbitan sesquiisostearate.

From the viewpoint of the dispersibility of the surface-treated powder and the feeling during use, the total content of the sorbitan fatty acid ester is preferably set in a range of 0.01% by mass to 20% by mass, more preferably set in a range of 0.01% by mass to 15% by mass, still more preferably in a range of 0.05% by mass to 10% by mass, and even still more preferably in a range of 0.1% by mass to 5% by mass of the total mass of the cosmetic. When the total content of the sorbitan fatty acid ester is 0.01% by mass or higher of the total mass of the cosmetic, the dispersibility of the surface-treated powder is enhanced, and there is a tendency that the temporal stability of the oil-in-water cosmetic is further improved, and, when the total content of the sorbitan fatty acid ester is 20% by mass or lower, there is a tendency that the emulsification dispersibility of the oil-in-water cosmetic is further improved.

The total content of the sorbitan fatty acid ester to the content of the surface-treated powder in terms of mass is preferably in a range of 0.0001 times to 150 times, more preferably in a range of 0.0005 times to 50 times, still more preferably in a range of 0.001 times to 5 times, even still more preferably in a range of 0.001 times to 1 time, and particularly preferably in a range of 0.001 times to 0.5 times. When the total content of the sorbitan fatty acid ester to the total content of the surface-treated powder in terms of mass is 0.0001 times or higher, the dispersibility of the surface-treated powder is enhanced, and there is a tendency that the temporal stability of the carotenoid is further improved, and, when the total content of the sorbitan fatty acid ester is 150 times or lower, there is a tendency that the dispersibility of the surface-treated powder is further improved.

### (Glycerin fatty acid ester)

Examples of the glycerin fatty acid ester include esters of glycerin and a linear or branched fatty acid having 8 to 20 carbon atoms in total. The glycerin fatty acid ester is more preferably an ester of glycerin and a linear or branched fatty acid having 10 to 18 carbon atoms in total. From the viewpoint of the temporal stability and the feeling during use of the oil-in-water cosmetic, the fatty acid capable of constituting the glycerin fatty acid ester together with glycerin is still more preferably a fatty acid selected from a group consisting of a linear unsaturated fatty acid, a branched saturated fatty acid, and a branched unsaturated fatty acid.

Examples of the fatty acid capable of constituting the glycerin fatty acid ester together with glycerin include caprylic acid, capric acid, lauric acid, myristic acid, palmitinic acid, stearic acid, behenic acid, oleic acid, linoleic acid, linolenic acid, isostearic acid, a castor oil fatty acid, arachidonic acid, and undecenoic acid. Among these, the fatty acid capable of constituting the glycerin fatty acid ester together with glycerin is preferably lauric acid, myristic acid, palmitinic acid, stearic acid, behenic acid, oleic acid, linoleic acid, linolenic acid, isostearic acid, in terms of the temporal stability and the feeling during use of the oil-in-water cosmetic.

The content of the glycerin fatty acid ester is preferably set in a range of 0.01% by mass to 20% by mass, more preferably in a range of 0.5% by mass to 10% by mass, and still more preferably in a range of 0.5% by mass to 5% by mass of the total mass of the cosmetic. When the content of the glycerin fatty acid ester is 0.01% by mass or higher of the total mass of the cosmetic, there is a tendency that the temporal stability of the oil-in-water cosmetic becomes more favorable, and, when the total content of the glycerin fatty acid ester is 20% by mass or lower, there is a tendency that the dispersibility of the surface-treated powder is further improved.

### (Fatty acid)

The oil-in-water cosmetic is capable of including a fatty acid. The inclusion of a fatty acid is capable of further improving the temporal stability and the feeling during use of the oil-in-water cosmetic.

The fatty acid may be either a saturated or unsaturated fatty acid and may be either a linear or branched fatty acid. The total number of carbon atoms in the fatty acid is not particularly limited, but is preferably in a range of 8 to 20 in total and more preferably in a range of 8 to 18 in total from the viewpoint of the temporal stability and the feeling during use of the oil-in-water cosmetic. Among these, from the viewpoint of the temporal stability and the feeling during use of the oil-in-water cosmetic, a fatty acid selected from a group consisting of branched saturated fatty acids and branched unsaturated fatty acids is preferred.

Examples of the fatty acid include caprylic acid, capric acid, lauric acid, myristic acid, palmitinic acid, stearic acid, behenic acid, oleic acid, linoleic acid, linolenic acid, isostearic acid, a castor oil fatty acid, arachidonic acid, and undecenoic acid. Among these, in terms of the temporal stability and the feeling during use of the oil-in-water cosmetic, examples thereof include lauric acid, myristic acid, palmitinic acid, stearic acid, behenic acid, oleic acid, linoleic acid, linolenic acid, and isostearic acid. Only one fatty acid may be used or a combination of two or more fatty acids may be used.

The content of the fatty acid is preferably set in a range of 0.01% by mass to 20% by mass, more preferably in a range of 0.5% by mass to 10% by mass, and still more preferably in a range of 0.5% by mass to 5% by mass of the total mass of the cosmetic. When the content of the fatty acid is 0.01% by mass or higher of the total mass of the cosmetic, there is a tendency that the temporal stability of the oil-in-water cosmetic becomes favorable, and, when the total content of the fatty acid is 20% by mass or lower, there is a tendency that the dispersibility of the surface-treated powder is improved.

### (e) Lipophilic thickener

In a case in which the oil-in-water cosmetic of the present invention includes the surface-treated powder in an oil phase, the oil-in-water cosmetic preferably includes a lipophilic thickener. The inclusion of the lipophilic thickener is capable of further improving the temporal stability of the oil phase in the oil-in-water cosmetic. When the lipophilic thickener coexists with the carotenoid in an oil phase, it is possible to further enhance the temporal stability of the carotenoid. The lipophilic thickener refers to a component that has an action of dispersing in an oil phase so as to hold an ultraviolet ray-scattering agent and/or water and stably assisting the dispersion thereof and, furthermore, is capable of imparting viscosity to the oil phase.

The form of the lipophilic thickener may be any of a particle form, a paste form, a slurry form, a gum form, a liquid form, a crystal form, and, from the viewpoint of the temporal stability of the cosmetic, the form of the lipophilic thickener is preferably a particle form, a paste form, or a slurry form and more preferably a particle form. Here, the particle form includes a spherical shape, a plate shape, an irregular form, which have the maximum diameter of 100 µm or smaller. The lipophilic thickener may be porous or have no pores therein.

In a case in which the lipophilic thickener has a particle form, the volume-average particle diameter of the lipophilic thickener is preferably in a range of 0.005 µm to 30 µm, more preferably in a range of 0.01 µm to 30 µm, and still more preferably in a range of 0.1 µm to 30 µm. When the volume-average particle diameter of the lipophilic thickener is 0.005 µm or larger, a user does not feel any roughness on the skin when using the oil-in-water cosmetic, and the feeling during use is further improved. When the volume-average particle diameter of the lipophilic thickener is 30 µm or smaller, there are no cases in which the surface area per unit weight of the lipophilic thickener becomes excessively low, and the adhesiveness to the skin tends not to be impaired. The volume-average particle diameter of the lipophilic thickener can be measured using a variety of commercially available particle size distribution meters.
For the measurement of the weight-average particle diameter of the lipophilic thickener, a dynamic light scattering method is preferably applied in consideration of the particle diameter range and ease of measurement. Examples of a commercially available determination device using dynamic light scattering include NANOTRAC UPA (manufactured by Nikkiso Co., Ltd.), a dynamic light scattering-type determination device of particle diameter distribution LB-550 (manufactured by Horiba Ltd.), and a denseness-based particle diameter analyzer FPAR-1000 (manufactured by Otsuka Electronics Co., Ltd.).

The volume-average particle diameter of the lipophilic thickener is a value measured using a denseness-based particle diameter analyzer FPAR-1000 (manufactured by Otsuka Electronics Co., Ltd.), and specifically, a value measured as described below can be employed.

That is, regarding the method for measuring the volume-average particle diameter of the lipophilic thickener, the lipophilic thickener is diluted with dimethicone so that the concentration thereof reaches 1% by mass, and the volume-average particle diameter is measured using a silica cell. The particle diameter of the lipophilic thickener can be obtained as the volume-average particle diameter when the refractive index of a specimen is set to 1.600, the refractive index of a dispersion medium is set to 1.000 (dimethicone), and the viscosity of the dispersion medium is set to the viscosity of dimethicone.

Examples of the lipophilic thickener include an organic denatured clay mineral, silica powder, porous silica powder, crosslinking silicone powder, polyamide powder, porous polyamide powder, polymethyl methacrylate powder, corn starch, and a dextrin fatty acid ester. Only one lipophilic thickener may be used or a combination of two or more lipophilic thickeners may be used.

Examples of the organic denatured clay mineral include dodecyl dimethyl benzyl ammonium montmorillonite clay and dimethyl dioctadecyl ammonium montmorillonite clay.

Examples of the silica powder include silica powder, silica silicate powder, silica dimethyl silylate powder, and silica dimethicone silylate powder.

Examples of the porous silica powder include porous silica powder, porous silica silylate powder, porous silica dimethyl silylate powder, and porous silica dimethicone silylate powder, and, from the viewpoint of stabilizing the viscosity, silica is preferred. Examples of commercially available products of the porous silica powder include SUNSPHERE H-31/H-32/H-33/H-51/H-52/H-53/H-121/H-122/H-201 (manufactured by AGC Si-Tech Co., Ltd.), VM-2270 Aerogel Fine Particle (manufactured by Dow Corning Toray Co., Ltd.), HDK (registered trademark) H2000, HDK (registered trademark) H15, HDK (registered trademark) H18, HDK (registered trademark) H20, and HDK (registered trademark) H30 (manufactured by Wacker Asahikasei Silicone Co., Ltd.), SYLOPURE (manufactured by Fuji Silysia Chemical Ltd.), TOKUSIL (manufactured by Tokuyama Corporation), and MICRO-BEADS SILICA GEL (manufactured by Fuji Davison Chemical Ltd.).

Examples of the crosslinking silicone powder include silicone gel powder, silicone rubber powder, silicone resin powder, and silicone elastomer, and these powders can be used without any distinction. From the viewpoint of the temporal stability of the cosmetic, the crosslinking silicone powder is preferably polymethylsilsesquioxane, an alkyl acrylate/dimethicone copolymer, a dimethicone/vinyl dimethicone crosspolymer, a dimethicone/bis-isobutyl PPG-20 crosspolymer, a dimethicone crosspolymer, a dimethicone (PET-10) crosspolymer, a PEG-10/lauryl dimethicone crosspolymer, a PEG-10 dimethicone/vinyl dimethicone crosspolymer, a dimethicone (PEG-10/15) crosspolymer, a PEG-15/lauryl dimethicone crosspolymer, a PEG-15/lauryl polydimethylsiloxyethyl dimethicone crosspolymer, a polyglyceryl-3/lauryl polydimethylsiloxyethyl dimethicone crosspolymer, a dimethicone/polyglycerin-3 crosspolymer, a lauryl dimethicone/polyglycerin-3 crosspolymer, a polyglyceryl-3/lauryl polydimethylsiloxyethyl dimethicone crosspolymer, a diphenyl dimethicone/vinyl diphenyl dimethicone/silsesquioxane crosspolymer, a dimethicone/bis-isobutyl PPG-20 crosspolymer, a dimethicone/vinyltrimethylsiloxysilicate crosspolymer, a dimethicone/phenyl vinyl dimethicone crosspolymer, a vinyl dimethicone/lauryl dimethicone crosspolymer, a vinyl dimethicone/methicone silsesquioxane crosspolymer, a lauryl polydimethylsiloxyethyl dimethicone/bis-vinyldimethicone crosspolymer.

Examples of a commercially available product of the silicone gel powder include KSG-15/16/1610, KSG-18A, KSG-41, KSP-100/101/102/105, KSP-300, 441/411, KSG-41/42/43/44, and KSG-240/310/320/330/340/710/320Z/350Z (manufactured by Shin-Etsu Chemical Co., Ltd.). Examples of a commercially available product of the silicone rubber powder include TORAYFIL E-506C, E-508, 9701 Cosmetic Powder, 9702 Powder, 9027/9040/9041/9045/9046/9041/9546 Silicone Elastomer Blend, EP-9215/EP-9216 TI/EP-9289 LL/EP-9293 AL, EL-8040 ID Silicone Organic Blend (manufactured by Dow Corning Toray Co., Ltd.), Wacker-Belsil (registered trademark) RG 100 (manufactured by Wacker Asahikasei Silicone Co., Ltd.), and NIKKOL SILBLEND-91 (manufactured by Nikko Chemicals Co., Ltd.).

Examples of the polyamide powder include nylon, nylon 6, nylon 11, nylon 12, a nylon 12/6/66 copolymer, nylon 6/12, and nylon 66. From the viewpoint of stabilizing the viscosity of the cosmetic, the polyamide powder is preferably nylon 6, nylon 12.

Examples of a commercially available product of the polyamide powder include TR-1, TR-2, SP-500 (manufactured by Toray Industries, Inc.), POMP 605, POMP 610 (manufactured by Ube Industries, Ltd.), and NYLON POWDER (manufactured by Nikko Rica Corporation).

Examples of the porous polyamide powder include nylon, porous nylon 6, porous nylon 11, porous nylon 12, a porous nylon 12/6/66 copolymer, porous nylon 6/12, and porous nylon 66.

Examples of the polymethyl methacrylate (hereinafter, also simply referred to as "PMMA") powder include, in addition to PMMA, powders of a methyl methacrylate crosspolymer, a methyl methacrylate/glycol dimethacrylate crosspolymer, a methyl methacrylate/acrylonitrile copolymer, a polymethyl methacrylate/dimethyl polysiloxane graft acrylic resin copolymer, and an ethylhexyl acrylate/methyl methacrylate copolymer. Examples of a commercially available product of PMMA include TECHPOLYMER (manufactured by Sekisui Chemical Co., Ltd.) and MATSUMOTO MICROSPHERE (manufactured by Matsumoto Yushi-Seiyaku Co., Ltd.).

As the corn starch, DRY FLO PURE (manufactured by Akzo Nobel N.V) can be used.

Examples of the dextrin fatty acid ester include dextrin palmitate, dextrin palmitate/2-ethyl hexanoate, dextrin myristate, dextrin stearate, dextrin plamitate/stearate, dextrin oleate, dextrin isopalmitate, and dextrin isostearate.

As the lipophilic thickener, a porous polymer made of styrene/stearyl methacrylate/divinyl benzene, a hydrogenated (styrene/isoprene) copolymer, a butylene/ethylene/styrene copolymer, a styrene/acrylamide copolymer, an ethylene/propylene/styrene copolymer, a clay mineral, bentonite, synthetic phlogopite, a polyamide resin, flaky titanium oxide, can also be used. Examples of a commercially available product of the lipophilic thickener include LUXELEN D (manufactured by Nihon Koken Kogyo Co., Ltd.), TIPAQUE CR-50 (manufactured by Ishihara Sangyo Kaisha, Ltd.), PIONEER GEL 12 PAO (manufactured by Hansen & Rosenthal KG), JOJOBA GLAZE HV/LV (manufactured by Nikko Chemicals Co., Ltd.), YODOSOL GH41F (manufactured by Akzo Nobel N.V), and KUNIPIA F/G (manufactured by Kunimine Industries Co., Ltd.).

From the viewpoint of the temporal stability of carotenoids, the temporal stability of the cosmetic, and improvement in the feeling of the cosmetic during use, the lipophilic thickener is preferably at least one lipophilic thickener selected from a group consisting of nylon 12, a dimethicone/vinyl dimethicone crosspolymer, polymethylsilsesquioxane, a vinyl dimethicone/methiconesilsesquioxane crosspolymer, and an alkyl acrylate/dimethicone copolymer and more preferably at least one lipophilic thickener selected from a group consisting of polymethylsilsesquioxane, nylon 12, a dimethicone/vinyl dimethicone crosspolymer, and a vinyl methicone/methiconesilsesquioxane crosspolymer.

In a case in which the surface-treated powder is included in an oil phase, the lipophilic thickener may be included in the oil phase including the surface-treated powder or may be included in an oil phase that is different from the oil phase including the surface-treated powder. In a case in which the lipophilic thickener is included in the oil phase including the surface-treated powder, there is an advantage of improving the dispersibility of the surface-treated powder. In a case in which the lipophilic thickener is included in an oil phase that is different from the oil phase including the surface-treated powder, there is an advantage of stabilizing the oil phase and stabilizing the entire cosmetic.

It is considered that at least one substance selected from a group consisting of nylon 12, a dimethicone/vinyl dimethicone crosspolymer, polymethylsilsesquioxane, a vinyl dimethicone/methiconesilsesquioxane crosspolymer, and an alkyl acrylate/dimethicone copolymer, particularly, at least one substance selected from a group consisting of polymethylsilsesquioxane, nylon 12, a dimethicone/vinyl dimethicone crosspolymer, and a vinyl methicone/methiconesilsesquioxane crosspolymer tends to further enhance both the temporal stability of carotenoids and the temporal stability of the oil-in-water cosmetic in a case in which the surface-treated powder is included in the same oil phase.

The total content of the lipophilic thickener in the oil-in-water cosmetic is preferably set in a range of 0.005% by mass to 30% by mass, more preferably set in a range of 0.01% by mass to 20% by mass, and still more preferably set in a range of 0.05% by mass to 15% by mass of the total mass of the cosmetic in terms of improving the temporal stability of the cosmetic. When the total content of the lipophilic thickener is 0.005% by mass or higher of the total mass of the cosmetic, there is a tendency that the dispersibility of the oil phase improves, and when the total content thereof is 30% by mass or lower, the feeling of the cosmetic during use is favorable when applied, and there is a tendency that the spreading of the cosmetic improves.

In a case in which the oil-in-water cosmetic includes the surface-treated powder in an oil phase, the total content of the lipophilic thickener is preferably in a range of 0.01% by mass to 9% by mass, more preferably in a range of 0.05% by mass to 9% by mass, and still more preferably in a range of 0.1% by mass to 5% by mass of the total mass of the cosmetic. When the content of the lipophilic thickener is set in the above-described range, it is possible to further improve a function of the surface-treated powder in the oil phase to temporally stabilize carotenoids.

The content of the lipophilic thickener to the content of the carotenoid in terms of mass is preferably in a range of 0.05 times to 300000 times, more preferably in a range of 1 time to 3000 times, and still more preferably in a range of 5 times to 300 times. When the content of the lipophilic thickener to the content of the carotenoid in terms of mass is 0.05 times or higher, the stability of carotenoids tends to be improved.

### (f) Water

The oil-in-water cosmetic of the present invention includes water.

The water that the oil-in-water cosmetic of the present invention includes is not particularly limited as long as the water can be applied to the cosmetic. The water in the cosmetic of the present invention is included as one of the components in a water-phase composition constituting a water phase.

The content of the water in the oil-in-water cosmetic of the present invention is preferably in a range of 1 % by mass to 90% by mass, more preferably in a range of 10% by mass to 80% by mass, and still more preferably in a range of 30% by mass to 70% by mass of the total mass of the cosmetic.

The content of the water is preferably in a range of 30% by mass to 95% by mass, more preferably in a range of 40% by mass to 90% by mass, and still more preferably in a range of 45% by mass to 85% by mass of the total mass of the water-phase composition.

### (g) Other components

The oil-in-water cosmetic of the present invention is capable of including, in addition to the above-described components, an oil-phase component such as a surfactant other than the sorbitan fatty acid ester and a water-phase component such as a water-soluble thickener as necessary.

### (Surfactant)

The oil-in-water cosmetic of the present invention is capable of including a surfactant other than the sorbitan fatty acid ester, the glycerin fatty acid ester, and the fatty acid as long as the effect of the present invention is not impaired.

The surfactant other than the sorbitan fatty acid ester (hereinafter, simply referred to as "surfactant" unless particularly otherwise described) may be either an ionic surfactant such as an anionic surfactant, a cationic surfactant, or an amphoteric surfactant or a nonionic surfactant. Examples of the ionic surfactant include alkylsulfonate salt, alkyl sulfate, monoalkyl phosphoate, and lecithin. Examples of the nonionic surfactant include an organic acid monoglyceride, a polyglycerin fatty acid ester, a propylene glycol fatty acid ester, a polyglycerin condensed ricinoleic acid ester, a sucrose fatty acid ester, a polyethylene glycol fatty acid ester, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerin fatty acid ester, a polyoxyethylene propylene glycol fatty acid ester, a polyoxyethylene castor oil, and a polyoxyethylene hardened castor oil. Among these, a polyglycerin fatty acid ester, for example, an ester of polyglycerin having a polarization degree in a range of 2 to 15 and a linear or branched fatty acid having 6 to 22 carbon atoms is more preferred from the viewpoint of improving the dispersibility of the surface-treated powder.

The content of the surfactant is preferably set in a range of 0.01% by mass to 30% by mass, more preferably in a range of 0.05% by mass to 20% by mass, and still more preferably in a range of 0.1% by mass to 10% by mass of the total mass of the cosmetic. When the content of the surfactant is 0.01% by mass or higher of the total mass of the cosmetic, there is a tendency that the temporal stability of the oil-in-water cosmetic becomes favorable, and, when the content thereof is 30% by mass or lower, there is a tendency that the feeling during use further improves.

### (Ultraviolet absorbents other than 4-tert-butyl-4-methoxy benzoyl methane)

The oil-in-water cosmetic is capable of including an ultraviolet absorbent other than the above-described 4-tert-butyl-4-methoxy benzoyl methane as long as the effect of the present invention is not impaired.

As the above-described ultraviolet absorbent, any of well-known oil-soluble or water-soluble ultraviolet absorbents can be used.

Examples of the oil-soluble ultraviolet absorbents include para-aminobenzoic acid, methyl para-aminobenzoate, glyceryl para-aminobenzoate, amyl para-dimethyl aminobenzoate, octyl para-dimethyl aminobenzoate, ethylene glycol salicylate, phenyl salicylate, octyl salicylate, butyl phenyl salicylate, homomentyl salicylate, octyl methoxycinnamate, ethoxyethyl methoxycinnamate, ethylhexyl methoxycinnamate, glyceryl monoethylhexanoate dimethoxycinnamate, hydroxymethoxybenzophenone, dihydroxy dimethoxybenzophenone, butyl methoxy benzoyl methane, and octyl triazone.

Examples of the water-soluble ultraviolet absorbent include benzophenone-based ultraviolet absorbents such as 2,4-dihydroxybenzophenone and 2,2'-dihydroxy-4-methoxybenzophenone; benzoimidazole-based ultraviolet absorbents such as phenylbenzoimidazole-5-sulfonic acid, salts thereof, phenylene bisbenzoimidazole tetrasulfonic acid, and salts thereof; urocanic acid, urocanic acid ethyl ester, 2,2-(1,4-phenylene)bis-(1H-benzimidazole-4,6-disulfonic acid), and terephthalylidene dicamphor sulfonic acid.

In terms of compensating ultraviolet ray-defending performance, the content of the ultraviolet absorbent other than 4-tert-butyl-4-methoxy benzoyl methane is preferably set in a range of 0.001% by mass to 30% by mass, more preferably in a range of 0.01% by mass to 20% by mass, and still more preferably in a range of 0.1% by mass to 10% by mass of the total mass of the cosmetic. In a case in which the ultraviolet absorbent other than 4-tert-butyl-4-methoxy benzoyl methane is used while not impairing the effect of the present invention, the proportion of other ultraviolet absorbents in 4-tert-butyl-4-methoxy benzoyl methane can be set in a range of 0.01% by mass to 10% by mass and can be set in a range of 0.1 % by mass to 8% by mass.

In terms of the feeling during use, the total amount of ultraviolet absorbents in the oil-in-water cosmetic is preferably set in a range of 0.001% by mass to 70% by mass, more preferably in a range of 0.01% by mass to 50% by mass, and still more preferably in a range of 0.1% by mass to 30% by mass of the total mass of the cosmetic.

### (Volatile oil component)

The oil-in-water cosmetic is capable of including a volatile oil component as a solvent. The inclusion of a volatile oil component as a solvent is capable of reducing a greasy feeling.

The volatile oil component refers to a component having a boiling point in a range of 60°C to 260°C at normal pressure. Examples of the volatile oil component used in the present invention include a volatile silicone-based oil and a hydrocarbon-based oil.

Examples of the volatile silicone-based oil include chain-like polysiloxanes such as dimethylpolysiloxane (1.5 cs [1.5x10⁻⁶ m²/s]), methyl phenyl polysiloxane, or methyl hydrogen polysiloxane, cyclic polysiloxane such as octamethyl cyclotetrasiloxane, cyclopentasiloxane, decamethylcyclopentasiloxane, dodecamethylcyclohexasiloxane, or tetramethyl tetrahydrogen cyclotetrasiloxane, and caprylyl methicone. Examples of a product thereof include KF96L-0.65, KF96L-1, KF96L-1.5, KF995 (manufactured by Shin-Etsu Chemical Co., Ltd.), SH200-1cs, SH200-1.5cs, SH200-2cs, 2-1184Fluid, SH245Fluid, DC246Fluid, DC345Fluid, SS3408 (manufactured by Dow Corning Toray Co., Ltd.), TSF404, TSF405, and TSF4045 (manufactured by Momentive Performance Materials Inc.).

As the volatile hydrocarbon-based oil, a volatile hydrocarbon-based oil selected from a group consisting of linear and branched volatile hydrocarbon-based oils may be used. Examples of the volatile hydrocarbon-based oil include C₈ to C₁₆ isoalkanes (also known as isoparaffins) such as isodecane, isododecane, and isohexadecane. Examples of a product thereof include ISOPAR (registered trademark) A, ISOPAR (registered trademark) C, ISOPAR (registered trademark) D, ISOPAR (registered trademark) E, ISOPAR (registered trademark) G, ISOPAR (registered trademark) H, ISOPAR (registered trademark) K, ISOPAR (registered trademark) L, and ISOPAR (registered trademark) M (manufactured by Exxon Mobil Corporation), SHELL SOL (registered trademark) 71 (manufactured by Royal Dutch Shell Plc), SALTROL (registered trademark) 100, SALTROL 130, and SALTROL 220 (manufactured by Koninklijke Philips N.V), ISOZOL (registered trademark) 400 (manufactured by Nippon Petrochemicals Co., Ltd.), PARLEAM (registered trademark) 4 (manufactured by NOF Corporation), and IP SOLVENT (registered trademark) 1620, IP SOLVENT (registered trademark) 2028 (manufactured by Idemitsu Petrochemical Co., Ltd.), ISOHEXADECANE, and TETRAISOBUTANE 90 (manufactured by Bayer AG), and PERMETHYL (registered trademark) 99A, PERMETHYL (registered trademark) 101A, and PERMETHYL (registered trademark) 102A (manufactured by Presperse LLC).

Only one volatile oil component may be used or a combination of two or more volatile oil components may be used.

In terms of the feeling during use, the content of the volatile oil component is preferably set in a range of 0.001% by mass to 60% by mass, more preferably in a range of 0.01% by mass to 40% by mass, and still more preferably in a range of 0.1% by mass to 20% by mass of the total mass of the cosmetic.

### (Polyhydric alcohol)

The oil-in-water cosmetic is capable of including a polyhydric alcohol. The inclusion of a polyhydric alcohol is capable of improving the feeling during use (moisture-retaining properties).

Examples of the polyhydric alcohol include glycerin, 1,3-butanediol, ethylene glycol, polysaccharides, reduced starch syrup, sucrose, erythritol, xylitol, glucose, galactose, sorbitol, maltotriose, and trehalose. Only one polyhydric alcohol can be used or a combination of two or more polyhydric alcohols can be used.

Since moisture-retaining properties can be imparted, the content of the polyhydric alcohol is preferably set in a range of 0.01% by mass to 20% by mass, more preferably in a range of 0.05% by mass to 10% by mass, and still more preferably in a range of 0.1% by mass to 10% by mass of the total mass of the cosmetic.

### (Other components)

The oil-in-water cosmetic is capable of including other components that are ordinarily used in cosmetics on the basis of use. Examples of the above-described components include a water-soluble organic solvent such as ethanol, a chelating agent, a whitening agent, an emollient such as isotridecyl isononanoate, a moisturizer, an antioxidant, a hydrophilic thickener such as agar or xanthan gum, a coloring agent, a preservative, a perfume, a variety of oily components, and a variety of aqueous components.

### (Manufacturing method)

The oil-in-water cosmetic can be manufactured using a manufacturing method in which an oil-phase composition including the above-described respective components and a water-phase composition are mixed together and the mixture is emulsified using an ordinary method.

When the surface-treated powder is formulated as the oil-phase component, the oil-in-water cosmetic can be manufactured by combining the surface-treated powder with other oil-phase components so as to prepare the oil-phase composition and combining the obtained oil-phase composition with the water-phase composition.

In a case in which the surface-treated powder is formulated as the water-phase component, the oil-in-water cosmetic can be manufactured by combining the surface-treated powder with other water-phase components so as to prepare the water-phase composition and combining the obtained water-phase composition with the oil-phase composition. Alternatively, the oil-in-water cosmetic can be manufactured by preparing a water-phase composition not including the surface-treated powder, combining the obtained water-phase composition with the oil-phase composition, preparing a preliminary oil-in-water composition not including the surface-treated powder, and adding the surface-treated powder to a water phase of the obtained preliminary oil-in-water composition. When the surface-treated powder is added to the water phase of the preliminary oil-in-water composition, the surface-treated powder may be singly added thereto, or it is also possible to combine the surface-treated powder with other water-phase components so as to prepare a water-phase composition and add the surface-treated powder as one component of the obtained water-phase composition.

In a case in which the oil-in-water cosmetic includes the surface-treated powder and the lipophilic thickener in the oil phase, the method for manufacturing the oil-in-water cosmetic is capable of further including a step of preparing an oil-phase composition including both the surface-treated powder and the lipophilic thickener or a step of respectively preparing an oil-phase composition including the surface-treated powder and another oil-phase composition including the lipophilic thickener in addition to the above-described step.

Regarding an emulsification method for obtaining the oil-in-water cosmetic in the form of an oil-in-water emulsified substance by combining the oil-phase composition and the water-phase composition, there is no particular limitation, and an ordinary method can be used.

When the emulsified substance is prepared, the ratio (mass) of the oil-phase composition to the water-phase composition is not particularly limited, but the oil phase/water phase ratio (% by mass) is preferably in a range of 0.1/99.9 to 50/50, more preferably in a range of 0.5/99.5 to 30/70, and still more preferably in a range of 1/99 to 20/80.

### (Form)

The form of the oil-in-water cosmetic is not particularly limited, and examples thereof include a skincare cosmetic such as a lotion, an emulsion, a cream, an eye cream, a serum, a massage material, a pack material, a spray, an ointment, or a body cream, a makeup cosmetic such as a makeup base, and a scalp cosmetic such as a leave-on essence.

The oil-in-water cosmetic of the present invention has a favorable ultraviolet ray shielding function and a favorable feeling during use and is thus particularly preferably used as a cosmetic for a sunblock.

### (Temporal stability of carotenoid)

The oil-in-water cosmetic of the present invention has high temporal stability of carotenoids. The temporal stability of carotenoids can be evaluated by irradiating a test specimen (10 g) with simulated sunlight with an intensity of 50 W/m² using a light resistance tester (Q-Sun Xe-3 manufactured by Q-LAB Corporation) at a distance of 20 cm for 120 minutes, and measuring the absorbance (480 nm) using a spectrophotometer.

Specifically, the absorbance of the test specimen before being irradiated with simulated sunlight, the absorbance of the test specimen irradiated with simulated sunlight for 5 minutes, and the absorbance of the test specimen irradiated with simulated sunlight for 120 minutes are respectively measured, and the percentage of the absorbance of the test specimen irradiated with simulated sunlight for 5 minutes or the absorbance of the test specimen irradiated with simulated sunlight for 120 minutes with respect to the absorbance of the test specimen before being irradiated with simulated sunlight is considered as the residual percentage of the carotenoid. When the residual percentage after 5 minutes of irradiation is 50% or higher and the residual percentage after 120 minutes of irradiation is 10% or higher, the temporal stability of carotenoids is evaluated to be high. Regarding the temporal stability of carotenoids in the oil-in-water cosmetic, it is preferable that the residual percentage after 5 minutes of irradiation is 60% or higher and the residual percentage after 120 minutes of irradiation is 30% or higher, and it is more preferable that the residual percentage after 5 minutes of irradiation is 70% or higher and the residual percentage after 120 minutes of irradiation is 50% or higher.

### (Temporal stability of cosmetic)

The oil-in-water cosmetic of the present invention preferably exhibits excellent temporal stability. The temporal stability of the oil-in-water cosmetic refers to the suppression of a change in the properties of the oil-in-water cosmetic for a certain period of time after the manufacture of the oil-in-water cosmetic. In the present invention, examples of the change in the properties include the aggregation of the surface-treated powder, and phase separation and/or gelation in the emulsified substance.

### (Feeling during use)

The oil-in-water cosmetic of the present invention exhibits a favorable feeling during use. In the present invention, the feeling during use refers to a fresh feeling when the cosmetic is applied and the absence of roughness after the cosmetic is applied. The fresh feeling when the cosmetic is applied refers to the occurrence of a change such as the separation of the emulsified substance and the oozing of water when the cosmetic is applied. The absence of the roughness after the cosmetic is applied refers to the absence of a tendency of the cosmetic being adhered to the skin so that a user can have substantially the same feeling in a portion to which the cosmetic is applied as if just water is applied instead of the cosmetic.

### Examples

Hereinafter, the present invention will be described in detail using examples. However, the present invention is not limited thereto.

### [Examples 1 to 4]

An oil-phase composition and a water-phase composition were prepared using an a component, a b component, a c component, a d component, an e component, an f component, and other components so that the final concentrations (% by mass) shown in Tables 1 and 2 below were obtained, and emulsification was carried out according to an ordinary method using the obtained oil-phase composition and water-phase composition, thereby obtaining an oil-in-water cosmetic. Individual components shown in Tables 1 and 2 will be described below in detail.

In each of Examples 1 and 2, the a component was mixed into cyclopentasiloxane using a disper, and other oil-phase components were combined with the mixture, thereby preparing an oil-phase composition. After that, the oil-phase composition and the water-phase composition were combined together at a ratio (mass ratio) of an oil phase to a water phase of 26:74, and emulsification was carried out using a homomixer under conditions of 80°C for 5 minutes at 5000 rpm, thereby obtaining an oil-in-water cosmetic. In the oil-in-water cosmetic of each of Examples 1 and 2, the a component is formulated into the oil phase.

In each of Examples 3 and 4, the a component was mixed into purified water and 1,3-butylene glycol (BG) using a disper, and other water-phase components were combined with the mixture, thereby preparing a water-phase composition. After that, the water-phase composition and the oil-phase composition were combined together at a ratio (mass ratio) of an oil phase to a water phase of 26:74, and emulsification was carried out using a homomixer under conditions of 80°C for 5 minutes at 5000 rpm, thereby obtaining an oil-in-water cosmetic. In the oil-in-water cosmetic of each of Examples 3 and 4, the a component is formulated into the water phase.

### [Comparative Examples 1 and 2]

Oil-in-water cosmetics were obtained in the same manner as in Example 1 except for the fact that, instead of the a component, silicone-coated titanium oxide and 4-tert-butyl-4-methoxy benzoyl methane (Comparative Example 1) or only silicone-coated titanium oxide (Comparative Example 2) were used, and silicone-coated titanium oxide and 4-tert-butyl-4-methoxy benzoyl methane, or silicone-coated titanium oxide were respectively formulated into the oil phase.

### [Comparative Examples 3 and 4]

Oil-in-water cosmetics were obtained in the same manner as in Example 3 except for the fact that, instead of the a component, silica-coated titanium oxide and 4-tert-butyl-4-methoxy benzoyl methane (Comparative Example 3) or only silica-coated titanium oxide (Comparative Example 4) were used, and silica-coated titanium oxide and 4-tert-butyl-4-methoxy benzoyl methane were respectively formulated into the water phase and the oil phase.

### [Examples 5 to 17 and Comparative Examples 5 and 6]

Oil-in-water emulsions were obtained in the same manner as in Example 1 except for the fact that the kinds and contents of individual components were changed as shown in Tables 3 and 4. Meanwhile, the individual components shown in Tables 3 and 4 will be described below in detail. In each of Examples 5 to 17 and Comparative Examples 5 and 6, the surface-treated powder is included in the oil phase.

### [Examples 18 to 31]

Oil-in-water emulsions were obtained in the same manner as in Example 1 except for the fact that, in the oil-in-water cosmetic of Example 1, an amount shown in Table 5 (0.5% by mass) of a comparative component shown in Table 6 was added instead of polymethylsilsesquioxane (the e component). In each of Examples 18 to 31, the surface-treated powder is included in the oil phase. Meanwhile, individual components shown in Tables 5 and 6 will be described below in detail.

### [Examples 32 to 39 and Comparative Examples 7 and 8]

Oil-in-water emulsions were obtained in the same manner as in Example 3 except for the fact that the kinds and contents of individual components were changed as shown in Tables 7 and 8. In Examples 32 to 39 and Comparative Examples 7 and 8, the surface-treated powder is included in the oil phase. Meanwhile, individual components shown in Tables 7 and 8 will be described below in detail.

### [Evaluations]

The following evaluations were carried out on individual test specimens of Examples 1 to 39 obtained above and individual test specimens of Examples 40 to 44 and Comparative Examples 1 to 8 described below. The results are shown in Tables 1, 3 and 4, and 6 to 8. In Table 1, "-'' in the rows regarding the evaluations of UV performances indicates that evaluation was not carried out. Meanwhile, regarding the comprehensive evaluations, test specimens with no C or D grades were evaluated to be "Pass", that is, to have no practical problems.

### (1) Temporal stability of carotenoid

Each test specimen (10 g) after the preparation of each of Examples 1 to 44 and Comparative Examples 1 to 8 was put into a glass-based dish with a diameter (φ) of 35 mm and was irradiated with simulated sunlight with an intensity of 50 W/m² using a light resistance tester (Q-Sun Xe-3 manufactured by Q-LAB Corporation) in an environment of 25°C at a distance of 20 cm for 120 minutes. The absorbance (480 mm) was measured using an integrating sphere in a spectrophotometer before the irradiation, after 5 minutes of the irradiation, and after 120 minutes of the irradiation, and the percentage of the absorbance after 5 minutes of the irradiation or the absorbance after 120 minutes of the irradiation with respect to the absorbance before the irradiation was considered as the residual percentage of the carotenoid after 5 minutes of irradiation or 120 minutes of irradiation. The temporal stability of carotenoids was evaluated on the basis of the obtained residual percentages of carotenoids using the following indexes.
AA: The residual percentage after 5 minutes of irradiation was 70% or higher, and the residual percentage after 120 minutes of irradiation was 50% or higher.
A: The residual percentage after 5 minutes of irradiation was in a range of 60% to lower than 70%, and the residual percentage after 120 minutes of irradiation was in a range of 30% to lower than 50%.
B: The residual percentage after 5 minutes of irradiation was in a range of 50% to lower than 60%, and the residual percentage after 120 minutes of irradiation was in a range of 10% to lower than 30%.
C: The residual percentage after 5 minutes of irradiation was in a range of 50% to lower than 60%, or the residual percentage after 120 minutes of irradiation was in a range of 10% to lower than 30%.
D: The residual percentage after 5 minutes of irradiation was lower than 50%, and/or the residual percentage after 120 minutes of irradiation was lower than 10%.

### (2) Dispersibility (initial stability of emulsified substance)

The test specimen (5 mg) of each of Examples 1 to 17, Examples 32 to 44, and Comparative Examples 1 to 8 was sandwiched between glass slides and was observed using an optical microscope (at a magnification of 350 times), and the presence or absence and emulsification state of an aggregate were visually evaluated using the following indexes.

Regarding the following indexes, in a case in which a plurality of 10 µm or larger black lumps could be observed in a single view, it is determined that "an aggregate is observed", in a case in which the sizes of the black lumps are in a range of 10 µm to 50 µm, it is determined that "a small aggregate is observed", and, in a case in which the sizes of the black lumps exceed 50 µm, it is determined that "a large aggregates is observed".

In addition, in a case in which, in a single view, oil droplets (emulsified particles) with (a long diameter of) shorter than 1 µm are evenly dispersed and other substances are not observed, the test specimen is determined to be in an "extremely homogeneous emulsification state as a whole", in a case in which oil droplets (emulsified particles) with (a long diameter of) shorter than 1 µm are evenly dispersed, the test specimen is determined to be in a "homogeneous emulsification state as a whole", and, in a case in which there are oil droplets (emulsified particles) with different sizes or oil droplets (emulsified particles) are significantly unevenly dispersed, the test specimen is determined to be in a "non-homogeneous emulsification state as a whole".
AA: An extremely homogeneous emulsification state not including any aggregates.
A: A homogeneous emulsification state not including any aggregates.
B: A homogeneous emulsification state including a small number of aggregates.
C: A non-homogeneous emulsification state including a small number of aggregates.
D: A large aggregate is observed.

### (3) Temporal stability of emulsified substance (gelation)

The test specimen (50 g) of each of Examples 1 to 44 and Comparative Examples 1 to 8 was put into a vial, and an acceleration test was carried out in a constant temperature bath at 50°C for one month. In order to evaluate the stability of the emulsified substance, in the specimen subjected to the test, the occurrence of gelation and phase separation was observed after one week from the initiation of the test (hereinafter, simply referred to as "after one week") and after one month from the initiation of the test (hereinafter, simply referred to as "after one month") respectively, and the temporal stability of the emulsified substance was evaluated using the following indexes.

The occurrence of gelation was evaluated by skimming the specimen using a spatula and visually observing whether the specimen had been solidified.

Phase separation was evaluated by visually observing the top surface of the specimen from the outside of the bottle and visually observing whether or not liquid oozed out from the top surface of the specimen or whether or not the specimen integrally flowed without being separated when the vial was inclined.
A: Gelation and phase separation were not observed after one week and after one month.
B: Gelation was not observed after one week and after one month. Phase separation was not observed after one week, but was observed after one month.
C: Both gelation and phase separation were not observed after one week, but were observed after one month.
D: Both gelation and phase separation were observed after one week.

### (4) Evaluation of ultraviolet ray shielding

On the basis of the in vitro measurement method of ISO24443, the specimen (32.5 mg) of each of Examples 1 to 4 and Comparative Examples 1 to 4 was applied onto a PMMA plate (HELIOPLATE HD6) using a finger with an even thickness, the transmittance was measured using an SPF analyzer (UV-2000S manufactured by Labsphere, Inc.), and the UVB and UVA-defending effect was evaluated using the following standards.

### (4-1) (Standards for evaluating UVB-depending effect)

A: The SPF value is 20 or higher.
B: The SPF value is in a range of 15 to lower than 20.
C: The SPF value is in a range of 10 to lower than 15.
D: The SPF value is lower than 10.

### (4-2) (Standards for evaluating UVA-depending effect)

A: The transmittance at 400 nm is lower than 40%.
B: The transmittance at 400 nm is in a range of 40% to lower than 70%.
C: The transmittance at 400 nm is in a range of 70% to lower than 90%.
D: The transmittance at 400 nm is 90% or higher.

### (5) Fresh feeling and absence of greasy feeling (feeling during use)

20 persons in an expert panel for evaluating the cosmetic were asked to use the test specimen (0.5 g) of each of Examples 1 to 44 and Comparative Examples 1 to 8, and a fresh feeling when the cosmetic was applied onto the face and the absence of roughness immediately after the cosmetic was applied and spread onto the entire face were evaluated using the following standards.
AA: Extremely favorable
A: Favorable
B: Slightly favorable
C: Normal
D: Poor

As the respective components shown in Tables 1 to 8, the following substances were used. Meanwhile, numerical values for the respective components in Tables 1 to 8 have a unit of % by mass.

### (Surface-treated powder)

- 4-Tert-butyl-4-methoxy benzoyl methane-coated titanium oxide: HXMT-10EXA, manufactured by TAYCA Corporation (primary average particle diameter: 10 µm)
- Silicone-coated titanium oxide: MTY-02, manufactured by TAYCA Corporation (primary average particle diameter: 10 µm)
- Silica-coated titanium oxide: MT-05, manufactured by TAYCA Corporation (primary average particle diameter: 10 µm)
- Astaxanthin: ASTOTS-S, manufactured by Takedashiki Co., Ltd.
- Sorbitan oleate: NIKKOL SO-10V, manufactured by Nikko Chemicals Co., Ltd.
- Sorbitan isostearate: NIKKOL SI-10RV, manufactured by Nikko Chemicals Co., Ltd.
- Sorbitan sesquioleate: NIKKOL SO-15V, manufactured by Nikko Chemicals Co., Ltd.
- Glyceryl stearate: NIKKOL MGS-AV, manufactured by Nikko Chemicals Co., Ltd.
- Polyglyceryl diisostearate-10: NIKKOL Decaglyn 2-ISV, manufactured by Nikko Chemicals Co., Ltd.
- Polymethylsilsesquioxane: KMP-590, manufactured by Shin-Etsu Chemical Co., Ltd.
- Dimethicone/vinyl dimethicone crosspolymer: BY29-129, manufactured by Dow Corning Toray Co., Ltd.
- Vinyldimethicone/methiconesilsesquioxane crosspolymer: KSP-100, manufactured by Shin-Etsu Chemical Co., Ltd.
- Butyl acrylate/glycol dimethacrylate crosspolymer, methyl methacrylate/glycol dimethacrylate crosspolymer: S-100, manufactured by Matsumoto Yushi-Seiyaku Co., Ltd. (volume-average particle diameter: 5 µm to 20 µm)
- Polyglyceryl-3/lauryl polydimethylsiloxyethyl dimethicone crosspolymer: KSG-850Z, manufactured by Shin-Etsu Chemical Co., Ltd.
- Dimethicone/(PEG-10/15) crosspolymer: KSG-210, manufactured by Shin-Etsu Chemical Co., Ltd.
- PEG-15/lauryl polydimethylsiloxyethyl dimethicone crosspolymer: KSG-350Z, manufactured by Shin-Etsu Chemical Co., Ltd.

**[Table 1]**

| | No. | Components | Example 1 | Comparative Example 1 | Comparative Example 2 | Example 2 | Example 3 | Comparative Example 3 | Comparative Example 4 | Example 4 |
|---|---|---|---|---|---|---|---|---|---|---|
| a | 1 | Surface-treated powder | | | | | | | | |
| | | Titanium oxide | 5 | | | 2.5 | 5 | | | 2.5 |
| | | 4-Tert-butyl-4-methoxy benzoyl methane | 1 | | | 0.5 | 1 | | | 0.5 |
| a | 2 | Surface-treated powder | | | | | | | | |
| | | Zinc oxide | | | | 2.5 | | | | 2.5 |
| | | 4-Tert-butyl-4-methoxy benzoyl methane | | | | 0.5 | | | | 0.5 |
| | 3 | Silicone-coated titanium oxide | | 5 | 5 | | | | | |
| | 4 | Silica-coated titanium oxide | | | | | | 5 | 5 | |
| | 5 | 4-Tert-butyl-4-methoxy benzoyl methane | | 1 | | | | 1 | | |
| b | 6 | Astaxanthin | 0.01 | 0.01 | 0.01 | 0.01 | 0.01 | 0.01 | 0.01 | 0.01 |
| c | 7 | Sodium acrylate/sodium acryloyldimethyl taurate copolymer | 0.4 | 0.4 | 0.4 | 0.4 | 0.4 | 0.4 | 0.4 | 0.4 |
| | | Common components 21 to 27 in Table 2 | 12.5 | 12.5 | 12.5 | 12.5 | 12.5 | 12.5 | 12.5 | 12.5 |
| | 8 | Isotridecyl isononanoate | 3 | 3 | 3 | 3 | 3 | 3 | 3 | 3 |
| | 9 | Cyclopentasiloxane | 15.5 | 15.5 | 16.5 | 15.5 | 15.5 | 15.5 | 16.5 | 15.5 |
| | 10 | Purified water | Residual amount | Residual amount | Residual amount | Residual amount | Residual amount | Residual amount | Residual amount | Residual amount |
| Evaluation items and determination results | | | | | | | | | | |
| (1)-1 | | Temporal stability of carotenoids, after 5 minutes of irradiation | 76% | 28% | 28% | 76% | 71% | 60% | 64% | 72% |
| (1)-2 | | Temporal stability of carotenoids, after 120 minutes of irradiation | 48% | 7% | 3% | 45% | 48% | 18% | 5% | 44% |
| (1) | | Temporal stability of carotenoids, comprehensive result | A | D | D | A | A | B | C | A |
| | (2) | Dispersibility | B | B | B | B | B | B | B | B |
| | (4-1) | UV performance UVB | A | B | - | B | A | C | - | B |
| | (4-2) | UV performance UVA | A | D | - | A | A | D | - | A |
| | (5) | Fresh feeling and absence of greasy feeling | A | B | B | A | A | C | C | A |

**[Table 2]**

| (mass%) | | |
|---|---|---|
| No. | Common components | Content |
| 21 | Polyglyceryl-10 diisostearate | 1.0 |
| 22 | BG | 1 |
| 23 | Methyl paraben | 0.2 |
| 24 | EDTA-2Na | 0.1 |
| 25 | Xanthan gum | 0.12 |
| 26 | Agar | 0.08 |
| 27 | Ethanol | 10 |

**[Table 3]**

| | No | Components | Example 1 | Example 5 | Example 6 | Example 7 | Comparative Example 5 | Comparative Example 6 |
|---|---|---|---|---|---|---|---|---|
| a | 1 | Surface-treated powder | | | | | | |
| | | Titanium oxide | 5 | 5 | 5 | 5 | 5 | 5 |
| | | 4-Tert-butyl-4-methoxy benzoyl methane | 1 | 1 | 1 | 1 | 1 | 1 |
| b | 2 | Astaxanthin | 0.01 | 0.01 | | 0.01 | 0.01 | 0.01 |
| | 3 | Lycopene | | | 0.01 | | | |
| c | 4 | Sodium acrylate/sodium acryloyldimethyl taurate copolymer | 0.4 | 0.4 | 0.4 | | | |
| | 5 | Ammonium acryloyldimethyl taurate/VP copolymer | | | | 0.4 | | |
| | 6 | Acrylates/alkyl acrylate (C10 to C30) crosspolymer to | | | | | 0.4 | |
| d | 7 | Sorbitan oleate | | *0.5* | *0.5* | *0.5* | *0.5* | *0.5* |
| | 8 | Sorbitan isostearate | | | | | | |
| | 9 | Sorbitan sesquioleate | | | | | | |
| | 10 | Glyceryl stearate | | | | | | |
| | 11 | Capric acid | | | | | | |
| | 12 | Oleic acid | | | | | | |
| | 13 | Isostearic acid | | | | | | |
| e | 14 | Polymethylsilsesquioxane | | | | | | |
| | | Common components 21 to 27 in Table 2 | 12.5 | 12.5 | 12.5 | 12.5 | 12.5 | 12.5 |
| | 15 | Isotridecyl isononanoate | | | | | | |
| | 16 | Cyclopentasiloxane | 15.5 | 15.0 | 15.0 | 15.0 | 15.0 | 15.4 |
| | 17 | Purified water | Residual amount | Residual amount | Residual amount | Residual amount | Residual amount | Residual amount |
| Evaluation items and determination results | | | | | | | | |
| (1) | | Temporal stability of carotenoids, comprehensive result | A | A | A | A | A | A |
| (2) | | Dispersibility | B | A | A | A | B | A |
| (3) | | Temporal stability of cosmetic | A | A | A | A | C | D |
| (5) | | Fresh feeling and absence of greasy feeling | A | A | A | A | C | B |

**[Table 4]**

| | No. | Components | Example 8 | Example 9 | Example 10 | Example 11 | Example 12 | Example 13 | Example 14 | Example 15 | Example 16 | Example 17 |
|---|---|---|---|---|---|---|---|---|---|---|---|---|
| a | 1 | Surface-treated powder | | | | | | | | | | |
| | | Titanium oxide | 5 | 5 | 5 | 5 | 5 | 5 | 5 | 5 | 5 | 5 |
| | | 4-Tert-butyl-4-methoxy benzoyl methane | 1 | 1 | 1 | 1 | 1 | 1 | 1 | 1 | 1 | 1 |
| b | 2 | Astaxanthin | 0.01 | 0.01 | 0.01 | 0.01 | 0.01 | 0.01 | 0.01 | 0.01 | 0.01 | 0.01 |
| | 3 | Lycopene | | | | | | | | | | |
| c | 4 | Sodium acrylate/sodium acryloyldimethyl taurate copolymer | 0.4 | 0.4 | 0.4 | 0.4 | 0.4 | 0.4 | 0.4 | 0.4 | 0.4 | 0.4 |
| | 5 | Ammonium acryloyldimethyl taurate/VP copolymer | | | | | | | | | | |
| | 6 | Acrylates/alkyl acrylate (C10 to C30) crosspolymer | | | | | | | | | | |
| d | 7 | Sorbitan oleate | 0.5 | | | | | | | 0.5 | 0.5 | 0.5 |
| | 8 | Sorbitan isostearate | | 0.5 | | | | | | | | |
| | 9 | Sorbitan sesquioleate | | | 0.5 | | | | | | | |
| | 10 | Glyceryl stearate | | | | 0.5 | | | | | | |
| | 11 | Capric acid | | | | | 0.5 | | | | | |
| | 12 | Oleic acid | | | | | | 0.5 | | | | |
| | 13 | Isostearic acid | | | | | | | 0.5 | | | |
| e | 14 | Polymethylsilsesquioxane | | | | | | | | 0.01 | 8.0 | 10.0 |
| | | Common components 21 to 27 in Table 2 | 12.5 | 12.5 | 12.5 | 12.5 | 12.5 | 12.5 | 12.5 | 12.5 | 12.5 | 12.5 |
| | 15 | Isotridecyl isononanoate | | | | | | | | | | |
| | 16 | Cyclopentasiloxane | 15.0 | 15.0 | 15.0 | 15.0 | 15.0 | 15.0 | 15.0 | 14.9 | 10.0 | 5.0 |
| | 17 | Purified water | Residual amount | Residual amount | Residual amount | Residual amount | Residual amount | Residual amount | Residual amount | Residual amount | Residual amount | Residual amount |
| Evaluation items and determination results | | | | | | | | | | | | |
| (1) | | Temporal stability of carotenoids, comprehensive result | A | A | A | A | A | A | A | AA | AA | AA |
| (2) | | Dispersibility | A | A | A | B | B | A | A | A | A | B |
| (3) | | Temporal stability of cosmetic | A | A | A | A | A | A | A | AA | AA | A |
| (5) | | Fresh feeling and absence of greasy feeling | A | A | A | A | A | A | A | AA | AA | A |

**[Table 5]**

| | No. | Components | Example 1 | Examples 18 to 31 |
|---|---|---|---|---|
| a | 1 | Surface-treated powder | | |
| | | Titanium oxide | 5 | 5 |
| | | 4-Tert-butyl-4-methoxy benzoyl methane | 1 | 1 |
| b | 2 | Astaxanthin | 0.01 | 0.01 |
| | 3 | Lycopene | | |
| c | 4 | Sodium acrylate/sodium acryloyldimethyl taurate copolymer | 0.4 | 0.4 |
| | 5 | Ammonium acryloyldimethyl taurate/VP copolymer | | |
| | 6 | Acrylates/alkyl acrylate (C10 to C30) crosspolymer | | |
| d | 7 | Sorbitan oleate | 0.5 | 0.5 |
| | 8 | Sorbitan isostearate | | |
| | 9 | Sorbitan sesquioleate | | |
| | 10 | Glyceryl stearate | | |
| e | | Comparative components in Table 6 | | 0.5 |
| | | Common components 21 to 27 in Table 2 | 12.5 | 12.5 |
| | 11 | Isotridecyl isononanoate | 3.0 | 3.0 |
| | 12 | Cyclopentasiloxane | 15.5 | 15.0 |
| | 13 | Purified water | Residual amount | Residual amount |

**[Table 6]**

| | Comparative components | Evaluation items | | |
|---|---|---|---|---|
| | | (1) | (3) | (5) |
| | | Temporal stability of carotenoids, comprehensive result | Temporal stability of cosmetic | Fresh feeling and absence of greasy feeling |
| Example 18 | Nylon-12 | AA | AA | AA |
| Example 19 | Dimethicone/vinyl dimethincone crosspolymer | AA | AA | AA |
| Example 20 | Vinyl dimethicone/methiconesilsesquioxane crosspolymer | AA | AA | AA |
| Example 21 | Alkyl acrylate/dimethicone copolymer | AA | AA | AA |
| Example 22 | Butyl acrylate/glycol dimethacrylate crosspolymer | AA | A | A |
| Example 23 | Methyl methacrylate/glycol dimethacrylate crosspolymer | AA | A | A |
| Example 24 | Methyl polymethacrylate | AA | A | A |
| Example 25 | Polyglyceryl-3/lauryl polydimethylsiloxyethyl dimethicone crosspolymer | AA | A | A |
| Example 26 | Vinyl dimethicone/lauryl dimethicone crosspolymer | AA | A | B |
| Example 27 | Dimethicone/polyglyceryl-3 crosspolymer | AA | A | B |
| Examples 28 | Lauryl dimethicone/polyglycerin-3 crosspolymer | AA | A | B |
| Example 29 | Dimethicone (PEG-10/15) crosspolymer | AA | A | B |
| Example 30 | PEG-15/lauryl polydimethylsiloxyethyl dimethicone crosspolymer | AA | A | B |
| Example 31 | Polyglyceryl- 3/lauryl polydimethylsiloxyethyl dimethicone crosspolymer | AA | A | B |

**[Table 7]**

| | No. | Components | Example 3 | Example 32 | Example 33 | Example 34 | Comparative Example 7 | Comparative Example 8 |
|---|---|---|---|---|---|---|---|---|
| a | 1 | Surface-treated powder | | | | | | |
| | | Titanium oxide | 5 | 5 | 5 | 5 | 5 | 5 |
| | | 4-Tert-butyl-4-methoxy benzoyl methane | 1 | 1 | 1 | 1 | 1 | 1 |
| b | 2 | Astaxanthin | 0.01 | 0.01 | | 0.01 | 0.01 | 0.01 |
| b | 3 | Lycopene | | | 0.01 | | | |
| c | 4 | Sodium acrylate/sodium acryloyldimethyl taurate copolymer | 0.4 | 0.4 | 0.4 | | | |
| | 5 | Ammonium acryloyldimethyl taurate/VP copolymer | | | | 0.4 | | |
| | 6 | Acrylates/alkyl acrylate (C10 to C30) | | | | | 0.4 | |
| | | crosspolymer | | | | | | |
| d | 7 | Sorbitan oleate | | 0.5 | 0.5 | 0.5 | 0.5 | 0.5 |
| | 8 | Sorbitan isostearate | | | | | | |
| | 9 | Sorbitan sesquioleate | | | | | | |
| | 10 | Glyceryl stearate | | | | | | |
| | 11 | Capric acid | | | | | | |
| | 12 | Oleic acid | | | | | | |
| | 13 | Isostearic acid | | | | | | |
| e | 14 | Polymethylsilsesquioxane | | | | | | |
| | | Common components 21 to 27 in Table 2 | 12.5 | 12.5 | 12.5 | 12.5 | 12.5 | 12.5 |
| | 15 | Isotridecyl isononanoate | 3.0 | 3.0 | 3.0 | 3.0 | 3.0 | 3.0 |
| | 16 | Cyclopentasiloxane | 15.5 | 15.0 | 15.0 | 15.0 | 15.0 | 15.4 |
| | 17 | Purified water | Residual amount | Residual amount | Residual amount | Residual amount | Residual amount | Residual amount |
| Evaluation items and determination results | | | | | | | | |
| (1) | | Temporal stability of carotenoids, comprehensive result | A | A | A | A | A | A |
| (2) | | Dispersibility | B | A | A | A | A | A |
| (3) | | Temporal stability of cosmetic | A | A | A | A | B | C |
| (5) | | Fresh feeling and absence of greasy feeling | A | A | A | A | C | B |

**[Table 8]**

| | No. | Components | Example 35 | Example 36 | Example 37 | Example 38 | Example 39 | Example 40 | Example 41 | Example 42 | Example 43 | Example 44 |
|---|---|---|---|---|---|---|---|---|---|---|---|---|
| a | 1 | Surface-treated powder | | | | | | | | | | |
| | | Titanium oxide | 5 | 5 | 5 | 5 | 5 | 5 | 5 | 5 | 5 | 5 |
| | | 4-Tert-butyl-4-methoxy benzoyl methane | 1 | 1 | 1 | 1 | 1 | 1 | 1 | 1 | 1 | 1 |
| b | 2 | Astaxanthin | 0.01 | 0.01 | 0.01 | 0.01 | 0.01 | 0.01 | 0.01 | 0.01 | 0.01 | 0.01 |
| b | 3 | Lycopene | | | | | | | | | | |
| c | 4 | Sodium acrylate/sodium acryloyldimethyl taurate copolymer | 0.4 | 0.4 | 0.4 | 0.4 | 0.4 | 0.4 | 0.4 | 0.4 | 0.4 | 0.4 |
| | 5 | Ammonium acryloyldimethyl taurate/VP copolymer | | | | | | | | | | |
| | 6 | Acrylates/alkyl acrylate (C10 to C30) crosspolymer | | | | | | | | | | |
| d | 7 | Sorbitan oleate | 0.5 | | | | | | | 0.5 | 0.5 | 0.5 |
| | 8 | Sorbitan isostearate | | 0.5 | | | | | | | | |
| | 9 | Sorbitan sesquioleate | | | 0.5 | | | | | | | |
| | 10 | Glyceryl stearate | | | | 0.5 | | | | | | |
| | 11 | Capric acid | | | | | 0.5 | | | | | |
| | 12 | Oleic acid | | | | | | 0.5 | | | | |
| | 13 | Isostearic acid | | | | | | | 0.5 | | | |
| e | 14 | Polymethylsilsesquioxane | | | | | | | | 0.01 | 8.0 | 10.0 |
| | | Common components 21 to 27 in Table 2 | 12.5 | 12.5 | 12.5 | 12.5 | 12.5 | 12.5 | 12.5 | 12.5 | 12.5 | 12.5 |
| | 15 | Isotridecyl isononanoate | 3.0 | 3.0 | 3.0 | 3.0 | 3.0 | 3.0 | 3.0 | 3.0 | 3.0 | 3.0 |
| | 16 | Cyclopentasiloxane | 15.0 | 15.0 | 15.0 | 15.0 | 15.0 | 15.0 | 15.0 | 14.9 | 10.0 | 5.0 |
| | 17 | Purified water | Residual amount | Residual amount | Residual amount | Residual amount | Residual amount | Residual amount | Residual amount | Residual amount | Residual amount | Residual amount |
| Evaluation items and determination results | | | | | | | | | | | | |
| (1) | | Temporal stability of carotenoids, comprehensive result | A | A | A | A | A | A | A | AA | AA | AA |
| (2) | | Dispersibility | A | A | A | B | B | A | A | A | A | B |
| (3) | | Temporal stability of cosmetic | A | A | A | A | A | A | A | AA | AA | A |
| (5) | | Fresh feeling and absence of greasy feeling | A | A | A | A | A | A | A | A | A | A |

As shown in Tables 1 to 8, the oil-in-water emulsified substances of Examples 1 to 44 obtained by combining 4-tert-butyl-4-methoxy benzoyl methane-coated titanium oxide or 4-tert-butyl-4-methoxy benzoyl methane-coated zinc oxide, astaxanthin or lycopene as the carotenoid, and a sodium acrylate/sodium acryloyldimethyl taurate copolymer or an ammonium acryloyldimethyl taurate/VP copolymer as a copolymer including acryloyldimethyl taurate as a copolymerization component all had favorable ultraviolet ray shielding functions and had favorable temporal stability of carotenoids and the feeling during use and could be evaluated to be "Pass" in the comprehensive evaluation.

In addition, it was found that, when the sorbitan fatty acid ester, the glycerin fatty acid ester, or the fatty acid was further combined therewith, it was possible to improve the dispersibility of the oil-in-water cosmetic (Examples 5 to 44). In addition, it was found that, when the lipophilic thickener was further combined therewith, there was a tendency that the temporal stability of carotenoids further improved (Examples 15 to 31). Particularly, it was found that, in a case in which the surface-treated powder and the lipophilic thickener were included in the same oil phase, there was a tendency that the temporal stability of the cosmetic further improved.

It was found that the above-described effects obtained from the examples could not be obtained in a case in which 4-tert-butyl-4-methoxy benzoyl methane was singly used (Comparative Examples 1 and 3), a case in which silicone-coated titanium oxide or silica-coated titanium oxide was used (Comparative Examples 2 and 4), and a case in which the copolymer including acryloyldimethyl taurate as a copolymerization component was not included (Comparative Examples 5 to 8).

### [Example 45] Milk cosmetic

A milk cosmetic was prepared using an ordinary method according to the following formulation. The following numerical values indicate % by mass of the total mass of the formulation. Meanwhile, 4-tert-butyl-4-methoxy benzoyl methane-coated titanium oxide is formulated in the same manner as in Example 1 and is thus included in an oil phase.

| <Composition> | <% by mass> |
|---|---|
| • Ethanol | 15.0 |
| • 1,3-Butylene glycol | 7.0 |
| • Ascorbic acid magnesium phosphate | appropriate amount |
| • Chelating agent | appropriate amount |
| • Glycerin | 3.0 |
| • Phenylbenzimidazole sulfonic acid | 1.0 |
| • Sodium acrylate/sodium acryloyldimethyl taurate copolymer | 0.8 |
| • 4-Tert-butyl-4-methoxy benzoyl methane-coated titanium oxide | 8.0 |
| • Astaxanthin | 0.005 |
| • Sorbitan sesquiisostearate | 0.6 |
| • Polyglyceryl-10 diisostearate | 0.7 |
| • Isostearic acid | 0.1 |
| • PEG/PPG-19/19 dimethicone | 1.5 |
| • Dimethicone | 4.0 |
| • Cyclopentasiloxane | 2.0 |
| • Pentaerythrityl tetraethylhexanoate | 3.0 |
| • Isodecane | 0.8 |
| • Trimethylsilyl siloxysilicate | 1.2 |
| • Ethylhexyl methoxycinnamate | 3.0 |
| • 2,4,6-Tris[4-(2-ethylhexyloxycarbonyl)anilino]-1,3,5-triazine | 0.1 |
| • Diethylamino hydroxybenzoyl hexyl benzoate | 0.2 |
| • Mica | 2.0 |
| • Kaolin | 1.0 |
| • Ethylhexylglycerin | 0.1 |
| • Phenoxyethanol | appropriate amount |
| • Purified water residual | amount |

### [Example 46] Liquid-type foundation

A liquid-type foundation was prepared using an ordinary method according to the following formulation. The following numerical values indicate % by mass of the total mass of the formulation. Meanwhile, 4-tert-butyl-4-methoxy benzoyl methane-coated titanium oxide is formulated in the same manner as in Example 1 and is thus included in an oil phase.

| <Composition> | <% by mass> |
|---|---|
| • Triethanolamine | 1.3 |
| • 1,3-Butylene glycol | 11.0 |
| • Methyl paraben | appropriate amount |
| • Xanthan gum | 0.01 |
| • 4-Tert-butyl-4-methoxy benzoyl methane-coated titanium oxide | 10.0 |
| • Astaxanthin | 0.02 |
| • Sorbitan sesquiisostearate | 1.2 |
| • Polysorbate 80 | 1.5 |
| • Castor oil fatty acid sucrose | 1.2 |
| • Glyceryl stearate | 0.5 |
| • Lecithin | 0.5 |
| • Sodium acrylate/sodium acryloyldimethyl taurate copolymer | 0.4 |
| • Isostearic acid | 2.0 |
| • Cetanol | 2.5 |
| • Squalane | 15.0 |
| • Methicone | 7.0 |
| • Ethylhexyl methoxycinnamate | 5.0 |
| • Methylene bis-benzotriazolyl tetramethylbutylphenol | 0.2 |
| Decyl glucoside | 0.1 |
| Pigment-grade titanium oxide | 10.0 |
| Stearyl glycyrrhetinate | 0.1 |
| Bengala | 0.05 |
| Yellow iron oxide | 0.6 |
| Black iron oxide | 0.2 |
| Purified water | residual amount |

### [Example 47] Sunblock lotion spray (filled with nitrogen)

A sunblock lotion spray (filled with nitrogen) was prepared using an ordinary method according to the following formulation. The following numerical values indicate % by mass of the total mass of the formulation. Meanwhile, 4-tert-butyl-4-methoxy benzoyl methane-coated titanium oxide is formulated in the same manner as in Example 1 and is thus included in an oil phase.

| <Composition> | <% by mass> |
|---|---|
| • Glycerin | 3.0 |
| • Ethanol | 5.0 |
| • Phenylbenzimidazole sulfonic acid | 2.0 |
| • Potassium hydroxide | appropriate amount |
| • Methyl paraben | appropriate amount |
| • Dipotassium glycyrrhizinate | 0.01 |
| • 1-Menthol | 0.05 |
| • Camphor | 0.02 |
| • 4-Tert-butyl-4-methoxy benzoyl methane-coated titanium oxide | 4.0 |
| • Lycopene | 0.0001 |
| • Sorbitan isostearate | 0.2 |
| • PEG-60 hydrogenated castor oil | 0.5 |
| • Sodium acrylate/sodium acryloyldimethyl taurate copolymer | 0.01 |

### [Example 48] Gel cosmetic

A gel cosmetic was prepared using an ordinary method according to the following formulation. The following numerical values indicate % by mass of the total mass of the formulation. Meanwhile, 4-tert-butyl-4-methoxy benzoyl methane-coated titanium oxide is formulated in the same manner as in Example 1 and is thus included in an oil phase.

| <Composition> | <% by mass> |
|---|---|
| • 1,3-Butylene glycol | 8.0 |
| • Glycerin | 2.0 |
| • Ethanol | 10.0 |
| • Xanthan gum | 0.1 |
| • Agar | 0.1 |
| • 4-Tert-butyl-4-methoxy benzoyl methane-coated titanium oxide | 7.0 |
| • Octocrylene | 2.0 |
| • Astaxanthin | 0.001 |
| • Sorbitan oleate | 0.4 |
| • Polyglyceryl-10 diisostearate | 0.4 |
| • Hydrogenated lecithin | 0.1 |
| • Sodium acrylate/sodium acryloyldimethyl taurate copolymer | 0.05 |
| • Polysilicone-15 | 0.1 |
| • Ethylhexyl triazone | 0.5 |
| • Nylon powder | 2.0 |
| • Perfume | appropriate amount |
| • Phenoxyethanol | appropriate amount |

As described above, according to the present invention, it is possible to provide an oil-in-water cosmetic having a favorable ultraviolet ray shielding effect, an excellent temporal stability of carotenoids, and a favorable feeling during use.

The disclosure of JP2013-103486 is incorporated in its entirety into the present specification for reference.

## Claims

1. An oil-in-water cosmetic comprising:
surface-treated powder including at least one oxide selected from the group consisting of titanium oxide and zinc oxide and having 4-tert-butyl-4-methoxy benzoyl methane on a surface;
at least one carotenoid; and
at least one copolymer including acryloyldimethyl taurate as a copolymerization component.

2. The oil-in-water cosmetic according to claim 1, further comprising: at least one compound selected from the group consisting of a sorbitan fatty acid ester, a glycerin fatty acid ester, and a fatty acid.

3. The oil-in-water cosmetic according to claim 1, further comprising at least one sorbitan fatty acid ester.

4. The oil-in-water cosmetic according to any one of claims 1 to 3, wherein the copolymer including acryloyldimethyl taurate as a copolymerization component is at least one copolymer selected from the group consisting of an ammonium acryloyldimethyl taurate/vinyl pyrrolidone copolymer, a sodium acrylate/sodium acryloyldimethyl taurate copolymer, and an ammonium acryloyldimethyl taurate/beheneth-25 methacrylate crosspolymer.

5. The oil-in-water cosmetic according to any one of claims 1 to 4, wherein the surface-treated powder is included in an oil phase.

6. The oil-in-water cosmetic according to claim 5, further comprising at least one lipophilic thickener, wherein the at least one lipophilic thickener is included in the oil phase including the surface-treated powder.

7. The oil-in-water cosmetic according to claim 6, wherein the lipophilic thickener is at least one lipophilic thickener selected from the group consisting of nylon-12, a dimethicone/vinyl dimethicone crosspolymer, polymethylsilsesquioxane, a vinyl dimethicone/methylsilsesquioxane crosspolymer, and an alkyl acrylate/dimethicone copolymer.

8. The oil-in-water cosmetic according to claim 6 or 7, wherein a total content of the at least one liphophilic thickener is in a range of 0.01% by mass to 9% by mass.

9. The oil-in-water cosmetic according to any one of claims 1 to 4, wherein the surface-treated powder is included in a water phase.

10. The oil-in-water cosmetic according to claim 1, wherein a content of the surface-treated powder in the oil-in-water cosmetic is from 0.1% by mass to 30% by mass.

11. The oil-in-water cosmetic according to claim 1, wherein a total content of the at least one copolymer including acryloyldimethyl taurate as a copolymerization component is from 0.01% by mass to 8% by mass, in terms of the amount of polymer (pure component), with respect to a total mass of the oil-in-water cosmetic.

## Patentansprüche

1. ÖI-in-Wasser-Kosmetikum aufweisend:
oberfächenbehandeltes Pulver, das mindestens ein Oxid umfasst, das ausgewählt ist aus der Gruppe, die aus Titanoxid und Zinkoxid besteht, und 4-tert-Butyl-4-methoxy-benzoyl-methan an einer Oberfläche hat;
mindestens ein Carotinoid; und
mindestens ein Copolymer, das Acryloyldimethyl-taurat umfasst, als eine Copolymerisationskomponente.

2. ÖI-in-Wasser-Kosmetikum nach Anspruch 1, außerdem aufweisend: mindestnes eine Verbindung, die ausgewählt ist aus der Gruppe, die aus einem Sorbitanfettsäureester, einem Glycerinfettsäureester und einer Fettsäure besteht.

3. ÖI-in-Wasser-Kosmetikum nach Anspruch 1, außerdem aufweisend mindestens einen Sorbitanfettsäureester.

4. ÖI-in-Wasser-Kosmetikum nach einem der Ansprüche 1 bis 3, bei dem das Copolymer, das Acryloyldimethyl-taurat als eine Copolymerisationskomponente umfasst, mindestens ein Copolymer ist, das ausgewählt ist aus der Gruppe, die aus einem Ammonium-acryloyldimethyl-taurat/Vinyl-pyrrolidon-Copolymer, einem Natriumacrylat/Natrium-acryloyldimethyl-taurat-Copolymer und einem Ammonium-acryloyldimethyl-taurat/Beheneth-25-methacrylat-Crosspolymer besteht.

5. Öl-in-Wasser-Kosmetikum nach einem der Ansprüche 1 bis 4, bei dem das oberflächenbehandelte Pulver in einer Ölphase enthalten ist.

6. Öl-in-Wasser-Kosmetikum nach Anspruch 5, außerdem mindestens ein lipophiles Verdickungsmittel aufweisend, wobei das mindestens eine lipophile Verdickungsmittel in der Ölphase enthalten ist, die das oberflächenbehandelte Pulver enthält.

7. Öl-in-Wasser-Kosmetikum nach Anspruch 6, bei dem das lipophile Verdickungsmittel mindestens ein lipophiles Verdickungsmittel ist, das ausgewählt ist aus der Gruppe, die aus Nylon-12, einem Dimethicon/Vinyl-dimethicon-Crosspolymer, Polymethylsilsesquioxan, einem Vinyl-dimethicon/methylsilsesquioxan-Crosspolymer und einem Alkylacrylat-dimethicon-Copolymer besteht.

8. Öl-in-Wasser-Kosmetikum nach Anspruch 6 oder 7, bei dem ein Gesamtgehalt des mindestens einen lipophilen Verdickungsmittels in einem Bereich von 0,01 Masse-% bis 9 Masse-% ist.

9. Öl-in-Wasser-Kosmetikum nach einem der Ansprüche 1 bis 4, bei dem das oberflächenbehandelte Pulver in einer Wasserphase enthalten ist.

10. Öl-in-Wasser-Kosmetikum nach Anspruch 1, bei dem ein Gehalt des oberflächenbehandelten Pulvers in dem Öl-in-Wasser-Kosmetikum von 0,1 Masse-% bis 30 Masse-% ist.

11. Öl-in-Wasser-Kosmetikum nach Anspruch 1, bei dem ein Gesamtgehalt des mindestens einen Copolymers, das Acryloyldimethyl-taurat als eine Copolymerisationskomponente enthält, bezogen auf die Menge an Polymer (Reinkomponente) 0,01 Masse-% bis 8 Masse-%, bezüglich einer Gesamtmasse des Öl-in-Wasser-Kosmetikums ist.

## Revendications

1. Produit cosmétique huile dans eau, comprenant :
une poudre ayant subi un traitement de surface, incluant au moins un oxyde sélectionné parmi le groupe consistant en de l'oxyde de titane et de l'oxyde de zinc et présentant du 4-tert-butyl-4-méthoxybenzoyl-méthane sur une surface ;
au moins un caroténoïde, et
au moins un copolymère incluant un taurate d'acryloyldiméthyle comme composant de copolymérisation.

2. Produit cosmétique huile dans eau selon la revendication 1, comprenant en outre : au moins un composé sélectionné parmi le groupe consistant en un ester d'acide gras de sorbitan, un ester d'acide gras de glycérine et un acide gras.

3. Produit cosmétique huile dans eau selon la revendication 1, comprenant en outre au moins un ester d'acide gras de sorbitan.

4. Produit cosmétique huile dans eau selon l'une quelconque des revendications 1 à 3, dans lequel le copolymère incluant le taurate d'acryloyldiméthyle comme composant de copolymérisation est au moins un copolymère sélectionné parmi le groupe consistant en un copolymère de taurate d'acryloyldiméthyle d'ammonium/vinylpyrrolidone, un copolymère d'acrylate de sodium/taurate d'acryloyldiméthyle de sodium, et un polymère réticulé de taurate d'acryloyldiméthyle d'ammonium/beheneth-25-méthacrylate.

5. Produit cosmétique huile dans eau selon l'une quelconque des revendications 1 à 4, dans lequel la poudre ayant subi un traitement de surface est incluse dans une phase huileuse.

6. Produit cosmétique huile dans eau selon la revendication 5, comprenant en outre un épaississant lipophile, dans lequel le au moins un épaississant lipophile est inclus dans la phase huileuse incluant la poudre ayant subi un traitement de surface.

7. Produit cosmétique huile dans eau selon la revendication 6, dans lequel l'épaississant lipophile est au moins un épaississant lipophile sélectionné parmi le groupe consistant en du nylon-12, un polymère réticulé de diméthicone/vinyldiméthicone, du polyméthylsilesquioxane, un polymère réticulé de diméthicone/méthylsilesquioxane, et un copolymère d'acrylate d'alkyle/diméthicone.

8. Produit cosmétique huile dans eau selon la revendication 6 ou 7, dans lequel une teneur totale du au moins un épaississant lipophile est comprise dans une plage allant de 0,01 % en masse à 9 % en masse.

9. Produit cosmétique huile dans eau selon l'une quelconque des revendications 1 à 4, dans lequel la poudre ayant subi un traitement de surface est incluse dans une phase aqueuse.

10. Produit cosmétique huile dans eau selon la revendication 1, dans lequel une teneur de la poudre ayant subi un traitement de surface dans le produit cosmétique huile dans eau est comprise dans une plage allant de 0,1 % en masse à 30 % en masse.

11. Produit cosmétique huile dans eau selon la revendication 1, dans lequel une teneur totale du au moins un copolymère incluant un taurate d'acryloyldiméthyle comme composant de copolymérisation est comprise dans une plage allant de 0,01 % en masse à 8 % en masse, en termes de la quantité de polymère (composant pur), par rapport à la masse totale de produit cosmétique huile dans eau.
